(19) Europäisches Patentamt
European Patent Office
Office européen des brevets

(11) **EP 2 693 893 B1**

(12) **FASCICULE DE BREVET EUROPEEN**

(45) Date de publication et mention de la délivrance du brevet:
**21.10.2015 Bulletin 2015/43**

(21) Numéro de dépôt: **12722748.6**

(22) Date de dépôt: **10.04.2012**

(51) Int Cl.:
*A23L 1/0562* (2006.01)     *A23J 3/08* (2006.01)
*A61K 38/38* (2006.01)     *C07K 14/76* (2006.01)
*A61Q 19/00* (2006.01)     *A61P 17/02* (2006.01)
*C09D 5/04* (2006.01)

(86) Numéro de dépôt international:
**PCT/FR2012/000138**

(87) Numéro de publication internationale:
**WO 2012/136909 (11.10.2012 Gazette 2012/41)**

(54) **HYDROGELS THIXOTROPES À BASE ALPHA-LACTALBUMINE, LEUR PROCÉDÉ DE PRÉPARATION ET LEURS UTILISATIONS**

THIXOTROPE ALPHA-LACTALBUMINHYDROGELE, HERSTELLUNGSVERFAHREN DAFÜR UND VERWENDUNGEN DAVON

THIXOTROPIC ALPHA-LACTALBUMIN HYDROGELS, METHOD FOR PREPARING SAME AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2011 FR 1153077**

(43) Date de publication de la demande:
**12.02.2014 Bulletin 2014/07**

(73) Titulaires:
 • **Commissariat à l'Énergie Atomique et aux Énergies Alternatives 75015 Paris (FR)**
 • **Centre National de la Recherche Scientifique 75016 Paris (FR)**

(72) Inventeurs:
 • **FORGE, Vincent F-38210 Vourey (FR)**
 • **MATHEVON, Carole 38360 SASSENAGE (FR)**
 • **PIGNON, Frédéric F-38000 Grenoble (FR)**

(74) Mandataire: **Bernstein, Claire Jacqueline Cabinet Orès 36, rue de Saint Pétersbourg 75008 Paris (FR)**

(56) Documents cités:
 EP-A1- 0 459 566     WO-A1-2008/130252
 WO-A2-2010/099185     US-A- 6 139 900

 • BLANCHET, C: "Repliement des prot ines et formation de fibres amyloides. Le cas de l Á-lactalbumine", INTERNET CITATION, 9 octobre 2008 (2008-10-09), pages 1-263, XP007919493, Extrait de l'Internet: URL:http://tel.archives-ouvertes.fr/docs/0 0/32/76/86/PDF/These_ finale.pdf cité dans la demande
 • JOHN GOERS ET AL: "Conformational Prerequisites for [alpha]-Lactalbumin Fibrillation +", BIOCHEMISTRY, vol. 41, no. 41, 1 octobre 2002 (2002-10-01), pages 12546-12551, XP055008205, ISSN: 0006-2960, DOI: 10.1021/bi0262698
 • GRAVELAND-BIKKER J F ET AL: "Unique milk protein based nanotubes: Food and nanotechnology meet", TRENDS IN FOOD SCIENCE AND TECHNOLOGY, ELSEVIER SCIENCE PUBLISHERS, GB, vol. 17, no. 5, 1 mai 2006 (2006-05-01), pages 196-203, XP025081434, ISSN: 0924-2244, DOI: 10.1016/J.TIFS. 2005.12.009 [extrait le 2006-05-01]
 • SUZANNE G. BOLDER ET AL: "Fibril Assemblies in Aqueous Whey Protein Mixtures", JOURNAL OF AGRICULTURAL AND FOOD CHEMISTRY, vol. 54, no. 12, 1 juin 2006 (2006-06-01), pages 4229-4234, XP055008208, ISSN: 0021-8561, DOI: 10.1021/jf060606s

- **DATABASE WPI Week 201116 Thomson Scientific, London, GB; AN 2010-Q12928 XP002660519, & CN 101 878 904 A (UNIV SOUTH CHINA TECHNOLOGY) 10 novembre 2010 (2010-11-10)**
- **Erinc Sahin ET AL: "Comparative effects of pH and ionic strength on protein-protein interactions, unfolding, and aggregation for IgG1 antibodies", Journal of Pharmaceutical Sciences, vol. 99, no. 12, 19 December 2010 (2010-12-19), pages 4830-4848, XP055179274, ISSN: 0022-3549, DOI: 10.1002/jps.22198**
- **Rong Zhou ET AL: "Utilization of Zwitterion-Based Solutions to Dissect the Relative Effects of Solution pH and Ionic Strength on the Aggregation Behavior and Conformational Stability of a Fusion Protein", Journal of Pharmaceutical Sciences, vol. 103, no. 10, 19 October 2014 (2014-10-19), pages 3065-3074, XP055179277, ISSN: 0022-3549, DOI: 10.1002/jps. 24118**
- **MAJHI PINAKI R ET AL: "Electrostatically driven protein aggregation: beta-lactoglobulin at low ionic strength", LANGMUIR, AMERICAN CHEMICAL SOCIETY, NEW YORK, NY; US, vol. 22, no. 22, 24 October 2006 (2006-10-24), pages 9150-9159, XP002604318, ISSN: 0743-7463, DOI: 10.1021/LA053528W [retrieved on 2006-09-21]**

Remarques:

Le dossier contient des informations techniques présentées postérieurement au dépôt de la demande et ne figurant pas dans le présent fascicule.

**Description**

La présente invention se rapporte à la préparation d'hydrogels présentant des propriétés avantageuses et utilisant un sous-produit de l'industrie laitière : l'α-lactalbumine, aux hydrogels ainsi obtenus et à leur utilisation, notamment pour la préparation de biomatériaux.

Les hydrogels produits dans le cadre de la présente invention présentent des caractéristiques rhéologiques particulières qui les rendent intéressants pour certaines applications ; en effet, il s'agit de gels thixotropes capables, sous une contrainte suffisante, de se déstructurer jusqu'à un état liquide puis de se restructurer une fois laissés au repos.

La protéine utilisée pour produire les hydrogels est l'α-lactalbumine (désignée a-La par la suite), en particulier, l'a-La d'origine bovine. Cette protéine laitière est retrouvée plus particulièrement dans le lactosérum ou petit-lait. Elle présente donc l'intérêt d'être une protéine naturelle et disponible en grandes quantités dans l'industrie fromagère, la fabrication du fromage étant réalisée à partir du « caillé » du lait (caséines précipitées et débarrassées du lactosérum).

Un gel est composé d'une molécule qui, en présence d'un solvant approprié, s'auto-assemble via des interactions chimiques ou physiques, et s'organise en un réseau. Les procédés spécifiques conduisant à la formation de gels dépendent des propriétés physico-chimiques et des interactions des composants du gel. Lorsque le solvant est de l'eau on parle d'hydrogel.

Un hydrogel est un réseau de chaînes polymères initialement solubles dans l'eau, mais qui sont devenues insolubles après réticulation. Les hydrogels sont des polymères naturels ou synthétiques super-absorbants (ils peuvent contenir plus de 99 % d'eau). Ils possèdent aussi un degré de flexibilité très similaire à celui des tissus naturels, à cause de leur importante teneur en eau. Leurs caractéristiques physiques, leur perméabilité ainsi que leur biocompatibilité en font d'excellents candidats comme biomatériaux utilisés pour des applications médicales incluant la délivrance de molécules ainsi que l'ingénierie tissulaire. En fonction de la nature des liaisons transversales, les hydrogels sont séparés en deux catégories : les gels chimiques issus des méthodes traditionnelles de réticulation ou « crosslinking » avec des liaisons covalentes et les gels physiques qui résultent de l'auto-assemblage de macromolécules (par exemple, glucidique ou protéique) et constituent des réseaux tenus ensemble par des enchevêtrements moléculaires et/ou des liaisons faibles (liaisons hydrophobes, ioniques, ponts hydrogène et forces de van der Waals) ; les interactions moléculaires de ces gels physiques peuvent être perturbées par des changements environnementaux tels que la température, le pH, la force ionique, la lumière ou même une molécule biologique donnée. La gélification est donc réversible en réponse à l'un ou plusieurs de ces stimuli.

La préparation d'hydrogels à partir de protéines du lait est déjà connue. Les protéines du lait sont des véhicules naturels pour les molécules bioactives grâce à leurs propriétés structurales et physicochimiques comme leur capacité à lier des ions et petites molécules ou encore leurs propriétés d'auto-assemblage et de gélification. Malgré la quantité de matériaux à base de protéines du lait (caséines ou protéines du lactosérum) déjà utilisés dans diverses industries, de nombreuses études sur ces protéines continuent et visent le développement de nouveaux nano-objets innovants : tels que des films alimentaires comestibles et biodégradables réalisés à partir de protéines du lait combinées avec d'autres biopolymères naturels (Chen 1995; Le Tien, Vachon et al. 2001) ; coacervats ou nano-vésicules de caséines ou d'autres protéines de lait qui ont notamment été développés comme systèmes de délivrance (Audic, Chaufer et al. 2003; Semo, Kesselman et al. 2007; Livney 2010) ; ou encore des gels de protéines du lait.

Parmi les gels de protéines de lait, on peut tout d'abord citer la gélification des caséines qui peut être obtenue sous différentes conditions : par l'acidité à pH 4,6 ; par l'action de la présure dont le principe actif est la chymosine, enzyme qui effectue un clivage protéolytique entraînant l'agrégation des micelles, et qui est utilisée pour l'encapsulation de bactéries probiotiques (Heidebach, Forst et al. 2009a) ; par l'action de la transglutaminase, enzyme qui ponte certains acides aminés entre eux (essentiellement acide glutamique et lysine) contribuant à la polymérisation des protéines et également utilisée pour la micro-encapsulation de cellules probiotiques (Heidebach, Forst et al. 2009b) ; par l'action de la génipine, agent de réticulation naturel des protéines, pour la délivrance contrôlée de molécules au niveau intestinal (contraction de l'hydrogel à pH acide (estomac) puis gonflement à pH neutre et donc libération du principe actif (intestin)) (Song, Zhang et al. 2009).

Il est également connu d'induire la gélification des protéines du lactosérum par élévation de la température (Paulsson, Hegg et al. 1986). Comme d'autres hydrogels, les gels réalisés à partir de protéines du lactosérum présentent un comportement de gonflement sensible au pH et utilisable pour la délivrance ciblée de molécules (Gunasekaran, Ko et al. 2007). Les gels de β-lactoglobuline, également induits par la température, sont très étudiés et ont été caractérisés à l'aide de diverses techniques telles que TEM (microscopie électronique à transmission), WAXS (wide angle X-ray scattering) et FTIR (Fourier transform infrared spectroscopy) (Kavanagh, Clark et al. 2000). La dénaturation des protéines peut entraîner des interactions hydrophobes entre elles, surtout la β-lactoglobuline (b-Lg) et la sérum albumine bovine (BSA) qui possèdent des thiols libres et peuvent donc s'inter-changer des ponts disulfures : par exemple, des nanosphères de BSA ont été réalisées avec une particule magnétique et/ou un photo-sensibilisateur et sont utilisables dans les traitements de cancer (Rodrigues, Simioni et al. 2009). Un procédé de gélification à froid a été récemment développé et rend les gels ainsi obtenus potentiellement intéressants pour la délivrance de molécules. L'intérêt de ces gels réside dans le fait que les bioactifs thermosensibles peuvent être ajoutés après le traitement thermique des lactosérums mais avant la gélification qui est ensuite induite par l'ajout de sels (calcium de préférence) ou par la diminution du pH.

De nombreuses mesures de rhéométrie ont été effectuées sur les gels de $\beta$-lactoglobuline seule (Gosal, Clark et al. 2004b) ou en mélange avec l'$\alpha$-lactalbumine (Kavanagh, Clark et al. 2000). Elles ont permis de déterminer les facteurs, pH et force ionique, affectant les caractéristiques physiques des gels (Loveday, Rao et al. 2009). Les gels de $\beta$-lactoglobuline (b-Lg) induits par une exposition prolongée à des températures élevées (80°C) se forment en deux temps et seraient essentiellement composés de polypeptides de $\beta$-lactoglobuline partiellement dégradée (Akkermans, Venema et al. 2008; Oboroceanu, Wang et al. 2010). Les gels de b-Lg peuvent aussi être obtenus par dissolution de la protéine dans un mélange particulier eau/alcool. Les caractéristiques rhéologiques et structurales de ces gels sont différentes (Gosal, Clark et al. 2004a; Gosal, Clark et al. 2004c; Loveday, Rao et al. 2009).

D'autres types de gels connus et réalisés à partir de protéines du lait sont formés à partir de nanotubes d'$\alpha$-lactalbumine (Ipsen, Otte et al. 2001). Ces nanotubes se forment par auto-assemblage de fragments d'$\alpha$-lactalbumine et sous certaines conditions (concentration en protéine minimale et rapport protéine/calcium) (Graveland-Bikker, Ipsen et al. 2004; Ipsen and Otte 2007). La particularité de ces fibres est qu'elles ne sont obtenues qu'en présence de calcium et avec une protéine qui a subi préalablement une protéolyse ménagée par une sérine protéase particulière, extraite de *Bacillus licheniformis* (Ipsen and Otte 2007).

La formation de gels à partir d'$\alpha$-lactalbumine intacte, et induite par de fortes températures (80°C), a été montrée à pH neutre dans deux articles étudiant notamment l'influence de l'$\alpha$-lactalbumine sur la gélification de la $\beta$-lactoglobuline (Hines and Foegeding 1993; Kavanagh, Clark et al. 2000). Plus particulièrement, l'équipe de Kavanagh *et al.* a suivi les gélifications à 80°C de différents rapports de concentration entre la b-Lg et l'a-La ainsi que des protéines seules constituant leurs témoins. Le témoin correspondant à l'a-La seule présente à pH 7 un temps de gélification 10 fois plus lent que celui observé avec la b-Lg seule. Ces gels d'a-La n'ont pas attiré l'attention des chercheurs et n'ont donc pas été caractérisés d'un point de vue structural et rhéologique. Un gel d'a-La préparé selon les conditions décrites par *Kavanagh et al.* a été préparé par la Demanderesse (voir l'exemple 3) ; il présente des caractéristiques macroscopiques différentes de celles des hydrogels mis au point par la Demanderesse ; il est dur, élastique et irréversible (il ne change pas de forme lorsqu'il est soumis à une contrainte).

Enfin, la thèse de C. Blanchet (« Repliement des protéines et formation de fibres amyloïdes. Le cas de l'$\alpha$-lactalbumine », soutenue le 23/06/2008) décrit un procédé de préparation de suspension d'$\alpha$-lactalbumine à 40°C, à pH 2 à différentes concentrations en sel (NaCl de 0 à 150 mM), ces procédés ne sont toutefois pas mis en oeuvre dans le but spécifique de préparer des gels mais pour caractériser le comportement de l'$\alpha$-lactalbumine.

Les gels obtenus avec 150 mM de NaCl ont toutefois été caractérisés, ils ont un comportement rhéofluidifiant ; il n'est pas suggéré dans cette thèse que de tels gels décrits puissent présenter un comportement thixotrope et un tel comportement ne peut se déduire des essais présentés dans cette thèse.

La Demanderesse a reproduit le procédé de préparation d'hydrogel d'$\alpha$-lactalbumine tel qu'il est décrit dans cette thèse et a pu confirmer expérimentalement que les gels ainsi obtenus ne sont pas thixotropes (voir l'exemple 4 ci-après).

Un fluide rhéofluidifiant (ou pseudoplastique) est un fluide dont la viscosité diminue si la contrainte de cisaillement ou la vitesse de déformation qui lui est appliquée augmente (voir le préambule de l'exemple 2).

Il convient de préciser que les fluides rhéofluidifiants ne sont pas nécessairement thixotropes, c'est le cas par exemple des gels de carbopol (Piau 2007; Tokpavi, Jay et al. 2009).

Dans le cadre de ses travaux, la Demanderesse est parvenue à mettre au point des hydrogels d'$\alpha$-lactalbumine rhéofluidifiants, à seuil et thixotropes.

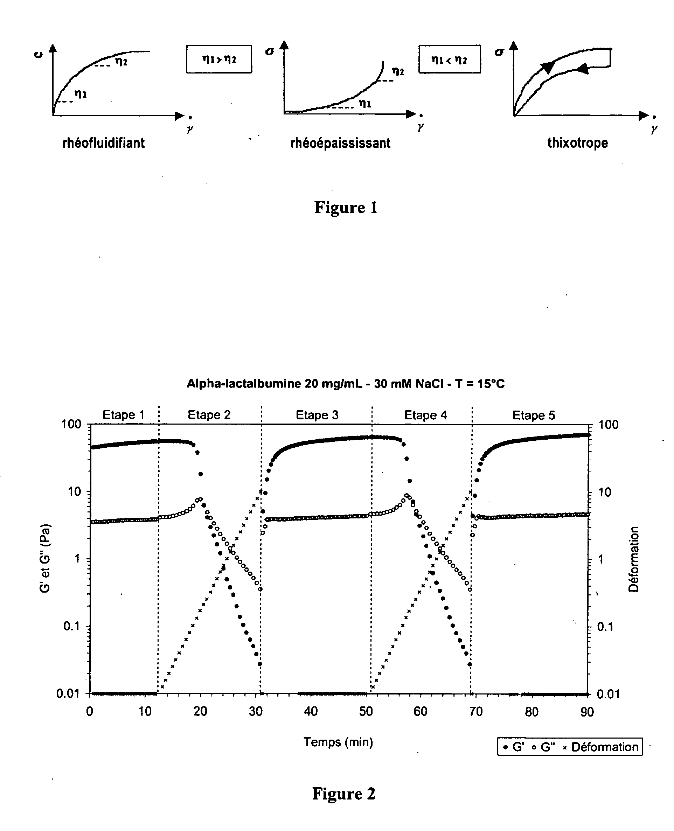

La définition de la thixotropie communément admise (Mewis 1979; Pignon, Magnin et al. 1998) est la suivante: un matériau est communément appelé thixotrope, si partant d'un état de repos pendant une période suffisamment longue, sa viscosité décroît avec le temps et sa structure se modifie, lorsqu'un gradient de cisaillement constant lui est appliqué. De manière réversible, si le cisaillement est interrompu, la viscosité croît de nouveau, le matériau recouvre alors graduellement la consistance et la structure qu'il avait au repos **(Figure 1).**

Cette caractéristique est très recherchée notamment pour l'étalement et l'application de nombreux produits en alimentaire (gels de protéines de lait ou de soja WO2008/130252), dans les peintures ou les produits cosmétiques. Elle peut également être très utile comme méthode non-invasive d'injection *in situ* d'hydrogels dans la délivrance ciblée de molécules ou pour la reconstruction tissulaire. Un article paru dans Nature Nanotechnology montre que des gels thixotropes composés par exemple de PEG-silice peuvent être utilisés et présentent divers avantages à la culture cellulaire 3D (Pek, Wan et al. 2008).

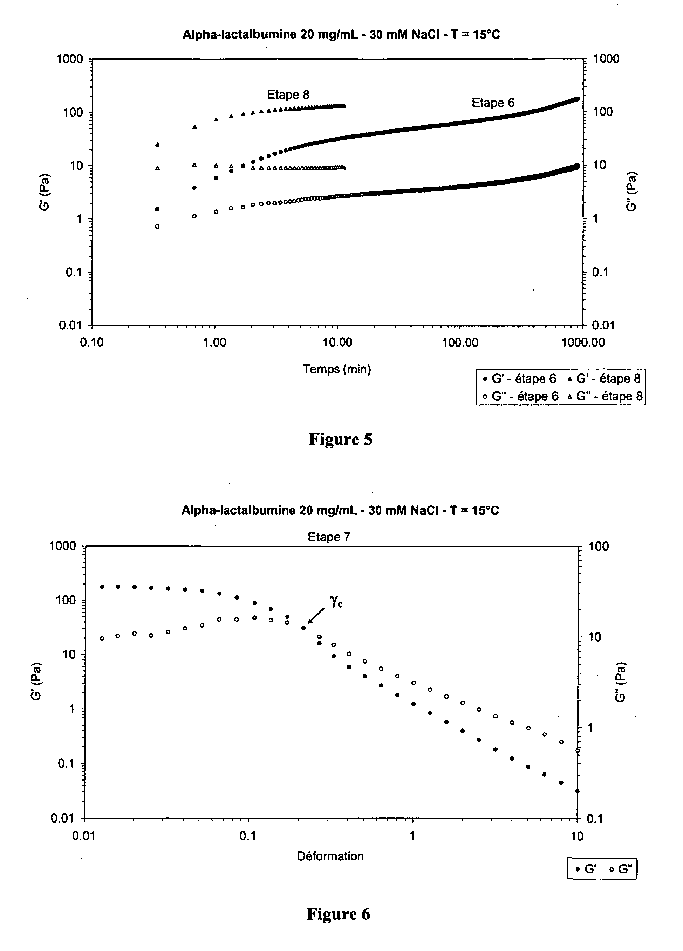

Les hydrogels selon l'invention sont dits à seuil car une contrainte minimum doit être appliquée pour permettre au matériau de couler au-delà d'une déformation, désignée déformation critique $\gamma_c$ ayant, dans le cadre de la présente invention, une valeur supérieure à 0,1, de préférence comprise entre 0,1 et 1. La déformation critique est atteinte lorsqu'une contrainte suffisamment élevée (contrainte seuil) est appliquée à un hydrogel complètement au repos pour que ledit hydrogel commence à couler ; elle est identifiée graphiquement par le croisement de la courbe du module de stockage G' et de celle du module de perte G" (voir l'exemple 2 et la **Figure 6**).

Plus particulièrement, la présente invention se rapporte à un procédé de préparation d'un hydrogel d'$\alpha$-lactalbumine à partir d'une suspension aqueuse d'$\alpha$-lactalbumine à une concentration $C_{a\text{-}La}$ comprise entre 5 et 60 mg/mL, comprenant les étapes suivantes :

a) la mise en suspension de l'$\alpha$-lactalbumine dans une solution aqueuse acide ayant une force ionique inférieure ou égale à 60 mM, de préférence, inférieure à 50 mM et, encore préférentiellement, ayant une valeur de 30 mM ; ladite mise en suspension consistant en :

(a1) la préparation d'une solution aqueuse acide ayant une concentration en protons exprimée en mM déterminée par la somme : (valeur numérique de $C_{a\text{-}La}$ exprimée en g/L) + 10 ;
(a2) la mise en suspension de l'$\alpha$-lactalbumine dans ladite solution aqueuse acide ; et
(a3) si nécessaire, l'ajustement du pH à une valeur comprise entre 1,5 et 2,5, de préférence, entre 1,8 et 2,2, et encore préférentiellement, le pH vaut 2,0 ;

b) la formation du gel à partir de ladite suspension d'$\alpha$-lactalbumine obtenue à l'issue de l'étape a) ; ladite formation du gel est mise en oeuvre dans les conditions suivantes :

- à une température inférieure à 60°C, de préférence comprise entre 35 et 55°C ;
- sous une agitation ayant une intensité définie par un nombre de Reynolds compris entre 37 et 1000, de préférence entre 300 et 500 ;
- pendant 10 heures à 1 semaine (168 heures), notamment, entre 48 et 96 heures, et
- en l'absence d'évaporation d'eau de ladite suspension d'$\alpha$-lactalbumine.

La suspension aqueuse d'$\alpha$-lactalbumine peut être également désignée solution dans ce qui suit.

De façon surprenante, malgré une force ionique faible ou nulle de la solution aqueuse utilisée pour la préparation de l'hydrogel d'a-La, la Demanderesse a constaté qu'il était tout de même possible d'obtenir un hydrogel thixotrope et que cet hydrogel présentait une stabilité dans le temps satisfaisante ; en effet, la Demanderesse a observé que les hydrogels à faible force ionique sont plus stables dans le temps que ceux obtenus à forte force ionique (> 60 mM) qui deviennent visqueux avec le temps. En outre, la préparation d'hydrogel ayant une teneur très faible en sel est avantageuse par exemple pour des utilisations comme texturant alimentaire.

L'$\alpha$-lactalbumine est une petite métalloprotéine globulaire de 14 kDa possédant 4 ponts disulfures, elle est structurellement homologue au lysozyme et nécessite la présence de calcium pour une structure fonctionnelle. C'est la protéine la plus abondante du lactosérum humain et la deuxième protéine après la b-Lg dans le lactosérum bovin. Elle fait partie des protéines modèles dans les études de repliement car elle est capable dans des conditions précises, d'adopter un état partiellement replié particulier : l'état « molten globule » (MG) (Permyakov and Berliner 2000).

L'a-La est la protéine du lait la moins allergène (Restani, Ballabio et al. 2009) et malgré une certaine résistance aux enzymes digestives en présence des autres protéines du lait, les peptides libérés par l'action de différentes protéases sur l'a-La sont largement étudiés.

De nombreux procédés d'enrichissement du lactosérum en a-La ou de purification de l'a-La sont utilisés à l'échelle industrielle : filtration sur membranes ; chromatographies sur colonnes (IEX, SEC, HIC) ; hydrolyse enzymatique pour dégrader les caséines ou la b-Lg, combinée avec de la filtration sur membrane ou encore précipitation isoélectrique combinée avec un traitement thermique pour précipiter l'a-La (Kamau, Cheison et al. 2010).

Le procédé selon l'invention peut être mis en oeuvre avec toute $\alpha$-lactalbumine ; selon un mode de mise en oeuvre particulier, il s'agit d'$\alpha$-lactalbumine issue de l'industrie fromagère provenant de lait de vache, de brebis, de chèvre, de bufflonne, de chamelle, de jument,...

Dans le cadre de la mise en oeuvre du procédé selon l'invention, la source d'$\alpha$-lactalbumine utilisée présente de préférence une pureté d'au moins 85%, préférentiellement d'au moins 90%. Il est également possible de mettre en oeuvre le procédé selon l'invention avec du lactosérum enrichi en $\alpha$-lactalbumine et ayant une teneur d'au moins 45% en poids d'$\alpha$-lactalbumine.

De préférence, la suspension d'$\alpha$-lactalbumine obtenue à l'issue de l'étape a) est filtrée. Cette filtration peut être réalisée avec un filtre ayant un seuil de coupure tel qu'il laisse passer les objets ayant un poids moléculaire inférieur ou égal à 20 kDa, comme les protéines d'a-La, mais retient les microorganismes et autres contaminants ; on peut ainsi utiliser des filtres de seuil de coupure inférieur ou égal à 0,5 $\mu$m, par exemple, les filtres commerciaux à 0,22 $\mu$m.

La mise en oeuvre de l'étape b) de formation du gel est réalisée avec une température homogène de l'ensemble de la suspension d'$\alpha$-lactalbumine.

L'agitation de la suspension d'$\alpha$-lactalbumine mise en oeuvre au cours de cette étape b) doit également être homogène dans tout le récipient d'agitation.

Afin de caractériser l'intensité de l'agitation, qui doit être faible mais non nulle, indépendamment de la géométrie et de la taille du récipient et de l'agitateur, la Demanderesse a déterminé une gamme de valeur du nombre de Reynolds adaptée à la mise en oeuvre du procédé selon l'invention ; ainsi, le nombre de Reynolds doit être compris entre 37 et 1000, de préférence, entre 300 et 500. Les modalités de détermination du nombre de Reynolds sont détaillées dans l'exemple 1 ci-après.

A titre d'exemple, lorsque le procédé est réalisé à l'échelle du laboratoire (volume de suspension d'α-lactalbumine compris entre 1 ml et 100 ml), l'agitation peut être effectuée à l'aide de plateau rotatif ou bien avec un barreau magnétique dont la longueur est comprise entre 70 et 90% du diamètre dudit récipient ; dans ces cas, la vitesse d'agitation est comprise entre 10 et 300 tours par minute (rpm).

Les gels d'a-La sont formés à pH acide, favorable à la forme MG et donc à la fibrillation de la protéine. La valeur du pH est extrêmement importante pour la formation des gels puisqu'elle est par exemple impossible à pH 3 (Kavanagh, Clark et al. 2000).

Lors de la mise en oeuvre de l'étape (a1) du procédé selon l'invention, la teneur en protons permettant d'obtenir le pH acide nécessaire à la formation de l'hydrogel peut être obtenue avec un acide fort, par exemple du HCl.

Il est essentiel de réaliser l'acidification selon étape (a1) avant l'étape (a2) de mise en suspension de l'a-La.

La force ionique mise en oeuvre pour la préparation de l'hydrogel selon l'invention et qui est comprise entre 0 et 60 mM est obtenue par l'ajout éventuel de sel pouvant être choisi parmi les halogénures de métal alcalin ou alcalinoterreux, tels que par exemple NaCl, KCl, $MgCl_2$, $CaCl_2$... ; les carbonates de métal alcalin ou alcalinoterreux ou leur mélange ; les phosphates, tels que par exemple le phosphate de sodium ou de potassium ou encore les sulfates tels que par exemple, le sulfate de sodium ou de magnésium....

La concentration en sel de la solution aqueuse d'a-La est classiquement déterminée par l'homme du métier en fonction de la force ionique souhaitée.

Selon une variante particulière de mise en oeuvre du procédé selon l'invention, il est mis en oeuvre sans ajout de sel.

L'exemple 2 qui suit montre la caractérisation rhéologique des hydrogels d'a-La obtenus par le procédé selon l'invention.

La présente invention se rapporte également aux hydrogels d'α-lactalbumine susceptibles d'être obtenus selon le procédé de l'invention.

Il s'agit plus spécifiquement d'hydrogels ayant une teneur en α-lactalbumine comprise entre 5 et 60 mg/mL ; un pH compris entre 1,5 et 2,5, de préférence entre 1,8 et 2,2 et encore préférentiellement, le pH vaut 2,0 ; une force ionique inférieure ou égale à 60 mM, de préférence, inférieure à 50 mM et, encore préférentiellement, ayant une valeur de 30 mM.

Ces hydrogels se caractérisent par un comportement de fluide rhéofluidifiant, à seuil de contrainte (un écoulement survient lorsqu'une déformation critique comprise entre 0,1 et 1 est appliquée) et thixotrope ; la viscosité de ces hydrogels diminue si la contrainte de cisaillement ou la vitesse de déformation qui leur est appliquée augmente, en outre, ces hydrogels se déstructurent sous l'influence du cisaillement (liquéfaction) et se restructurent lorsque le cisaillement cesse (solidification).

Produits à partir de sources naturelles comme le lait ou le lactosérum, les hydrogels d'α-lactalbumine selon l'invention s'inscrivent dans le cadre du développement durable. Le produit est naturel et donc biodégradable, biocompatible, non toxique et considéré comme GRAS (generally recognized as safe). De plus, le procédé de production est compatible avec les procédés de chimie verte, c'est-à-dire chimie douce qui préserve l'environnement puisqu'il n'utilise ni solvant, ni agent de réticulation.

Les protéines du lait, et en particulier l'α-lactalbumine, présentent des propriétés remarquables :

- leurs propriétés physico-chimiques sont bien connues : structure, stabilité, solubilité, affinité pour les métaux, hydrophobicité...
- l'auto-assemblage des protéines se fait sous forme de fibres, une des fondations des nanotechnologies ;
- elles ont de hautes valeurs nutritionnelles ;
- elles présentent divers effets biologiques observés surtout à partir des peptides dérivés de leur protéolyse (Madureira, Pereira et al. 2007).

En outre, l'utilisation de ces protéines à l'échelle industrielle est adaptée car :

- leur source, le lactosérum, a un faible coût ;
- les procédés de purification des protéines du lactosérum à l'échelle industrielle sont déjà connus ;
- les quantités disponibles sont considérables : d'une part du fait de la quantité d'a-La contenue dans le lactosérum (plus de 1 g/L dans le lactosérum bovin) et d'autre part du fait des quantités de lactosérum disponibles puisque sous-produit de l'industrie fromagère et donc matière à valoriser.

Le lactosérum ou petit-lait est le principal déchet des fromageries et des caséineries. Les principales protéines du lactosérum sont la β-lactoglobuline (b-Lg), l'α-lactalbumine (a-La), des immunoglobulines, la sérum albumine bovine (SAB) et la lactoferrine (Lf).

Les quantités de lactosérum disponibles dans le monde sont considérables. En France, 15 milliards de litres de lactosérum sont générés chaque année par la production des fromages de vache. En l'absence de solution de valorisation, le lactosérum non autoconsommé par le bétail est déversé dans les rivières ou épandu dans les champs, avec des conséquences néfastes pour le milieu naturel : pollution des cours d'eau, lacs et nappes phréatiques et nuisances olfactives. Cette pollution est principalement due à la fermentation des matières organiques du lactosérum (lactose et matières azotées) et à la diminution de la teneur en oxygène dissous de l'eau au-dessous d'un seuil acceptable. En effet, les demandes chimique et biologique en oxygène (DCO et DBO) de ce déchet sont fortes (DCO de 50 à 70 g/L) et en font une matière gravement polluante. La mise en place de dispositifs économiquement acceptables, visant à collecter et valoriser le lactosérum, est aujourd'hui obligatoire pour réduire ces atteintes à l'environnement.

La forte valeur nutritionnelle, fonctionnelle et biologique de l'a-La justifie son intérêt dans les domaines alimentaire, pharmaceutique et cosmétique. L'utilisation des hydrogels d'a-La selon l'invention peut être envisagée sous forme d'hydrogels en tant que tels bien évidemment mais aussi sous forme de films par étalement (spin-coating) puis séchage des gels, ou encore sous forme de fils par extrusion ou électro-spinning des gels.

La Demanderesse a mis en évidence que les hydrogels d'a-La ayant une teneur en $\alpha$-lactalbumine comprise entre 5 et 60 mg/mL et préparés à pH acide, c'est-à-dire, compris entre 1,5 et 2,5, de préférence entre 1,8 et 2,2 et encore préférentiellement, à pH 2,0, quelle que soit leur force ionique, sont rhéofluidifiants, à seuil et thixotropes.

Bien que ces hydrogels présentent un pH acide à l'issue de leur préparation, il est possible d'augmenter leur pH afin de l'adapter aux utilisations qu'on souhaite faire desdits hydrogels tout en préservant leurs propriétés rhéologiques.

Ainsi, selon un autre de ses objets, la présente invention se rapporte à des hydrogels thixotropes ayant une teneur en $\alpha$-lactalbumine comprise entre 5 et 60 mg/mL et réparés à un pH acide mais dont le pH a pu être augmenté par la suite, pour une utilisation où leurs propriétés rhéologiques s'avèrent avantageuses.

Composés de produits comestibles, les hydrogels d'a-La trouvent en particulier de nombreuses applications dans le domaine de l'industrie agro-alimentaire.

Ainsi, la présente invention se rapporte à un produit alimentaire comprenant un hydrogel thixotrope d'$\alpha$-lactalbumine ayant une teneur en $\alpha$-lactalbumine comprise entre 5 et 60 mg/mL et à l'utilisation d'un tel hydrogel d'$\alpha$-lactalbumine comme agent texturant alimentaire. Selon un mode de réalisation particulier, la force ionique desdits hydrogels est comprise entre 0 et 60 mM, de préférence, inférieure à 50 mM et, encore préférentiellement, ayant une valeur de 30 mM.

Selon la consistance souhaitée du produit alimentaire, il pourra contenir entre 0,5 et 98% d'hydrogel thixotrope d'$\alpha$-lactalbumine en poids par rapport au poids total dudit produit alimentaire.

De par les propriétés de l'$\alpha$-lactalbumine rappelées plus haut, les hydrogels selon l'invention sont particulièrement adaptés à la préparation de biomatériaux. On entend par "biomatériaux" des matériaux destinés à être en contact temporaire ou permanent avec différents tissus, organes ou fluides d'un être vivant, dans un but diagnostique, préventif ou thérapeutique ; les biomatériaux comprennent également les matériaux destinés à être implantés dans un organisme vivant.

Ainsi, la présente invention se rapporte également aux hydrogels thixotropes d'$\alpha$-lactalbumine ayant une teneur en $\alpha$-lactalbumine comprise entre 5 et 60 mg/mL, pour une utilisation comme produit utilisé pour le traitement et/ou la cicatrisation des plaies ; en particulier, ces hydrogels ont une force ionique comprise entre 0 et 60 mM. En effet, les hydrogels d'$\alpha$-lactalbumine répondent aux critères généralement considérés pour la préparation de pansement : biocompatibilité et absence de cytotoxicité ; prévention de la déshydratation de la plaie avec le maintien d'un environnement humide ; protection contre les poussières et les bactéries ; maintien des échanges gazeux ; application facile sur la plaie et facilité pour l'enlever après cicatrisation.

En outre, afin d'améliorer leur efficacité, les hydrogels pourront également comprendre un ou plusieurs composés actifs tels que des composés capables de promouvoir la cicatrisation cutanée, par exemple en favorisant l'épithélialisation en délivrant des molécules actives spécifiques (ex : EGF) ou des composés antimicrobiens.

Ainsi la présente invention se rapporte à un pansement composé d'au moins un hydrogel thixotrope d'$\alpha$-lactalbumine ayant une teneur en $\alpha$-lactalbumine comprise entre 5 et 60 mg/mL ; selon une variante particulière, ces hydrogels ont une force ionique comprise entre 0 et 60 mM, de préférence, inférieure à 50 mM et, encore préférentiellement, ayant une valeur de 30 mM. Optionnellement, ledit pansement comprend en outre au moins un composé actif tel qu'un agent cicatrisant ou un agent antimicrobien.

Selon une variante, le pansement selon l'invention comporte un film externe perméable à l'air et imperméable aux liquides et aux microorganismes ; un tel film peut par exemple être composé de polyuréthane.

Selon un autre de ses objets, la présente invention se rapporte à une composition cosmétique comprenant au moins un hydrogel thixotrope d'$\alpha$-lactalbumine ayant une teneur en $\alpha$-lactalbumine comprise entre 5 et 60 mg/mL, et à l'utilisation d'au moins un tel hydrogel thixotrope d'α-lactalbumine pour la préparation d'une composition cosmétique, en particulier, comme agent hydratant de la peau dans des compositions destinées au soin de la peau (gel, crème, lotion..) ou comme tensio-actif (produits lavant, shampoing...) ; selon une variante particulière, ces hydrogels ont une force ionique comprise entre 0 et 60 mM, de préférence, inférieure à 50 mM et, encore préférentiellement, ayant une valeur de 30 mM.

Enfin, de par leur comportement thixotrope, les hydrogels ayant une teneur en α-lactalbumine comprise entre 5 et 60 mg/mL sont avantageusement utilisés pour la préparation de peintures, en particulier industrielles. En effet, les hydrogels permettent à ces peintures de rester solides lors de leur transport et leur stockage évitant ainsi un écoulement indésirable tout en présentant un étalement facile à l'utilisation. En outre, les hydrogels permettent d'améliorer la stabilité et la conservation des peintures industrielles qui sont habituellement utilisées dans des tanks agités en permanence. Par une agitation réalisée uniquement au moment de leur utilisation, ils permettent en plus de réaliser une économie énergétique.

Ainsi, la présente invention se rapporte encore à l'utilisation d'un hydrogel thixotrope d'α-lactalbumine ayant une teneur en α-lactalbumine comprise entre 5 et 60 mg/mL, pour la préparation de peintures, en particulier industrielles, et à des peintures comprenant un tel hydrogel ; selon une variante particulière, ces hydrogels ont une force ionique comprise entre 0 et 60 mM, de préférence, inférieure à 50 mM et, encore préférentiellement, ayant une valeur de 30 mM.

Outre les dispositions qui précèdent, l'invention comprend encore d'autres dispositions qui ressortiront de la description qui va suivre, qui se réfèrent à des exemples de mise en oeuvre de la présente invention, ainsi qu'aux figures annexées dans lesquelles :

Figures

La **Figure 1** illustre le comportement des fluides thixotropes dont la viscosité décroît avec le temps lorsqu'un gradient de cisaillement constant lui est appliqué, et qui, de manière réversible, croît de nouveau si le cisaillement est interrompu.

La **Figure 2** représente le suivi de la déstructuration et de la restructuration à 15°C au cours du temps d'un hydrogel d'a-La à 20 mg/mL et 30 mM de NaCl en petites déformations en cisaillement harmonique. Différentes procédures de suivi de l'évolution des modules élastiques G' (cercle plein) et visqueux G" (cercle vide) en fonction du temps et de la déformation imposée (croix), ont été appliquées ; les contraintes appliquées à chaque étape sont détaillées dans l'exemple 2 qui suit.

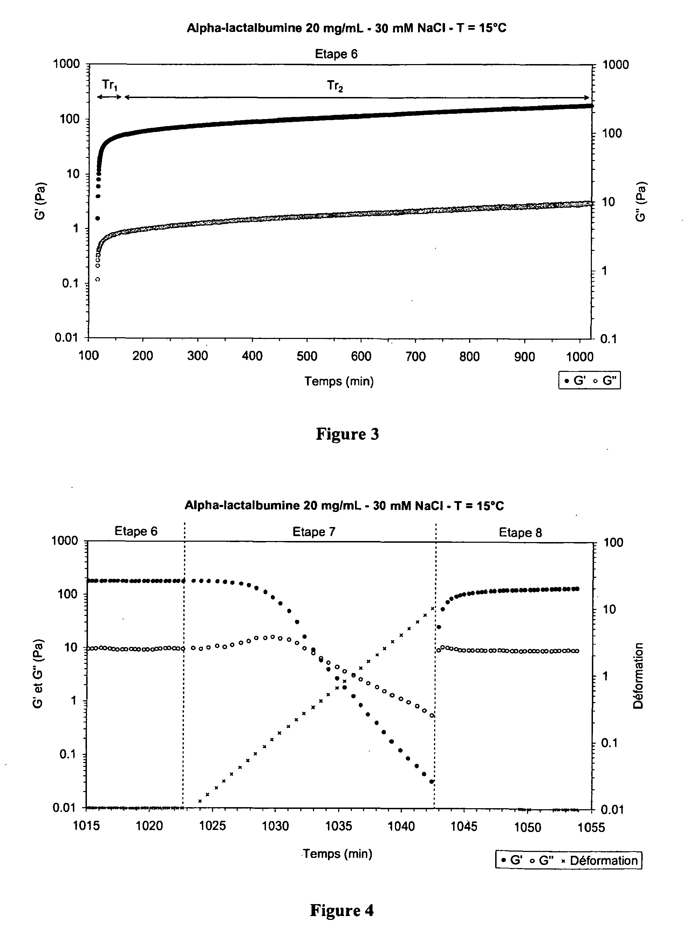

La **Figure 3** représente le suivi de la restructuration à 15°C au cours du temps d'un hydrogel d'a-La à 20 mg/mL et 30 mM de NaCl après une déstructuration en grande déformation à un gradient de cisaillement de 10 s$^{-1}$ ; le module élastique G' est représenté en cercle plein et le module visqueux G" en cercle vide.

La **Figure 4** illustre la fin du suivi de la restructuration à 15°C au cours du temps d'un hydrogel d'a-La à 20 mg/mL et 30 mM de NaCl après une déstructuration en grande déformation à un gradient de cisaillement de 10 s$^{-1}$ (étape 6), puis l'étape 7 de déstructuration en cisaillement harmonique à amplitude de déformation croissante, et enfin l'étape 8 de suivi de la reprise de consistance en petite déformation

La **Figure 5** est une comparaison de la cinétique de restructuration à 15°C temps d'un hydrogel d'a-La à 20 mg/mL et 30 mM de NaCl (étape 6) après une déstructuration en grande déformation à un gradient de cisaillement de 10 s$^{-1}$, (G' est représenté en triangle plein et G" en triangle vide) et (étape 8) après une déstructuration en petites déformations en cisaillement harmonique à amplitude de déformation croissante (G' est représenté en cercle plein et G" en cercle vide).

La **Figure 6** représente le suivi de la déstructuration en cisaillement harmonique à amplitude de déformation croissante à 15°C temps d'un hydrogel d'a-La à 20 mg/mL et 30 mM de NaCl : identification de la déformation critique $\gamma_c$ d'environ 0.2 au delà de laquelle la transition sol-gel commence à apparaître (étape 7).

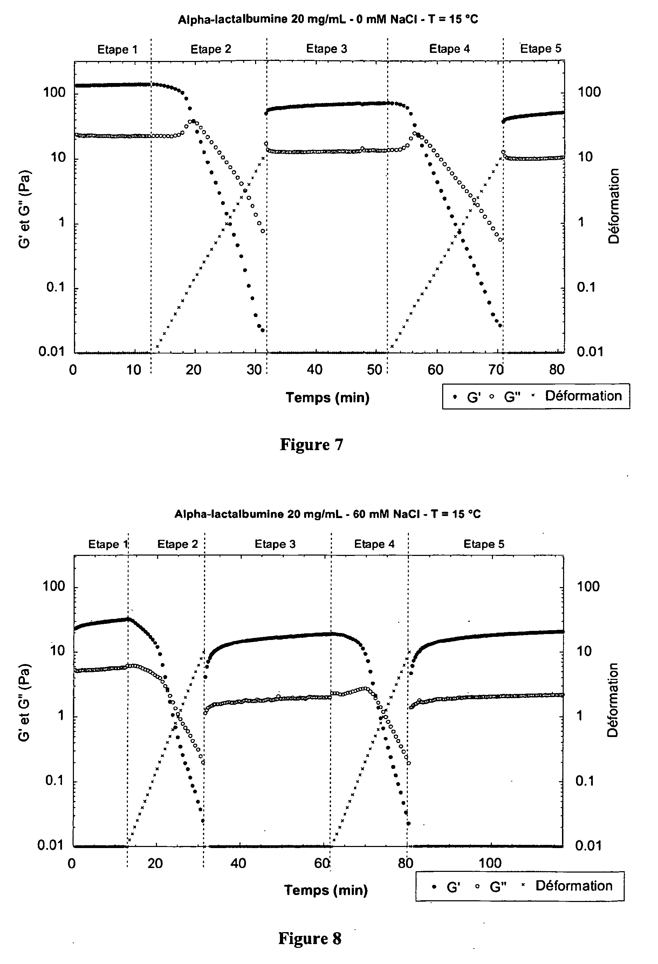

La **Figure 7** représente le suivi de la déstructuration et de la restructuration à 15°C au cours du temps d'un hydrogel d'a-La en petites déformations en cisaillement harmonique ; 20 mg/mL - 0 mM NaCl.

La **Figure 8** représente le suivi de la déstructuration et de la restructuration à 15°C au cours du temps d'un hydrogel d'a-La en petites déformations en cisaillement harmonique ; 20 mg/mL - 60 mM NaCl

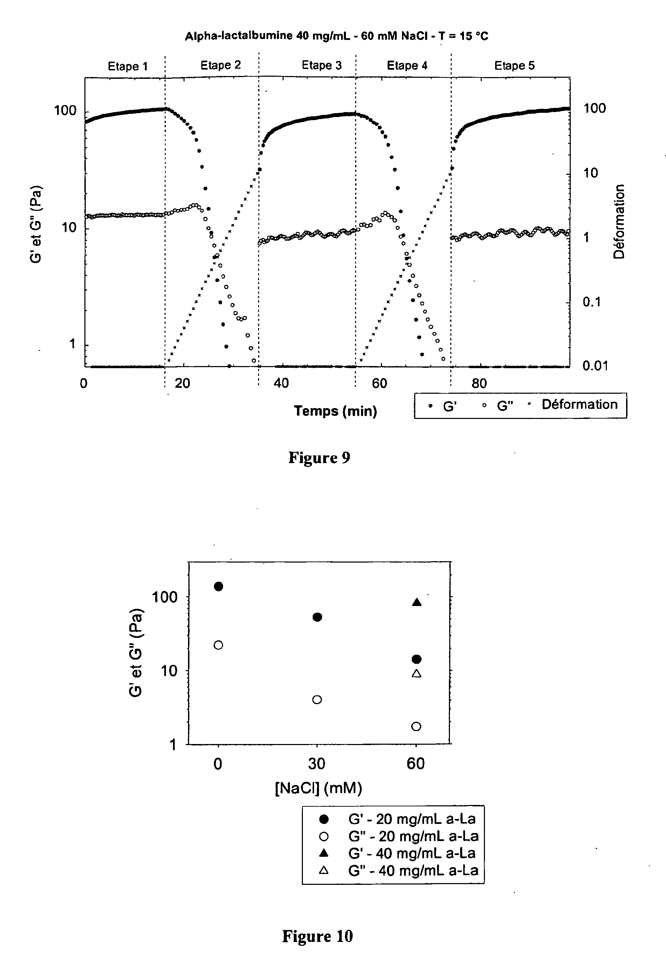

La **Figure 9** représente le suivi de la déstructuration et de la restructuration à 15°C au cours du temps d'un hydrogel d'a-La en petites déformations en cisaillement harmonique ; 40 mg/mL - 60 mM NaCl.

La **Figure 10** compare les niveaux des modules viscoélastiques G' et G" en fonction de la force ionique en NaCl et de la concentration en a-La : le module G' pour un hydrogel à 20 mg/mL d'a-La est représenté par des disques pleins, le module G" pour un hydrogel à 20 mg/mL d'a-La est représenté par des disques vides, le module G' pour un hydrogel à 40 mg/mL d'a-La est représenté par des triangles pleins et le module G' pour un hydrogel à 40 mg/mL d'a-La est représenté par des triangles vides. Les niveaux ont été comparés au même temps de restructuration 500 s de l'étape 3 correspondant à la zone de cinétique lente de restructuration.

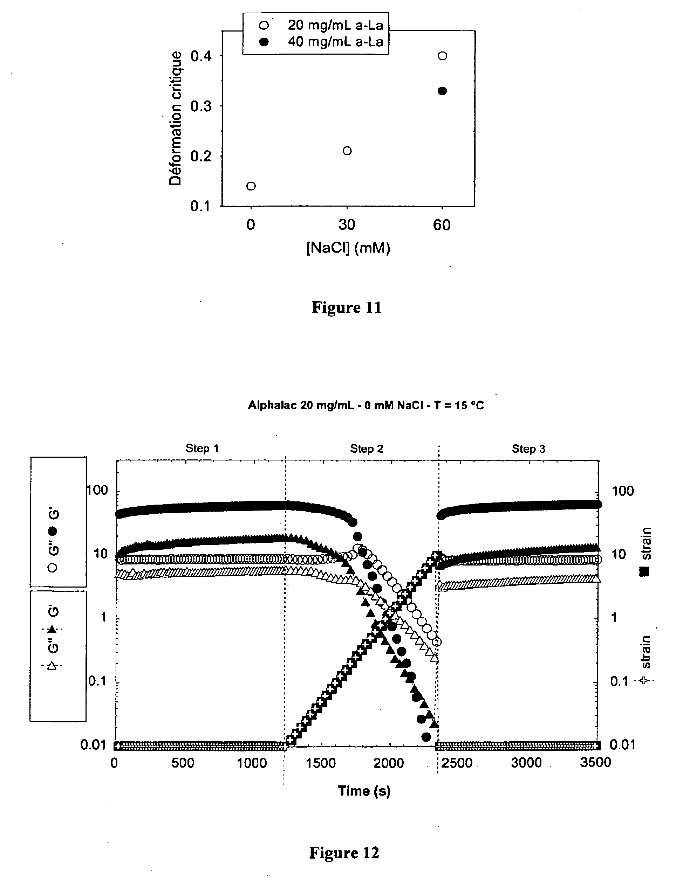

La **Figure 11** est un graphe qui compare les niveaux de déformation critique $\gamma_c$ en fonction de la force ionique en NaCl et de la concentration en a-La (disques vides pour une concentration en a-La de 20 mg/mL et disques pleins pour une concentration en a-La de 40 mg/mL). Pour chaque condition, le phénomène de déstructuration est très reproductible ce qui met en évidence la grande capacité du système à subir des sollicitations de cisaillement diverses sans subir de modification physico-chimique ou de dénaturation quelconque.

La **Figure 12** représente le suivi de la déstructuration et de la restructuration à 15°C au cours du temps de deux hydrogels d'a-La à 20 mg/mL et 0 mM de NaCl (l'un préparé à partir d'a-La purifié, le second à partir de lactosérum enrichi à 45% d'a-La) en petites déformations en cisaillement harmonique. Différentes procédures de suivi de l'évolution des modules élastiques G' (cercle plein pour l'hydrogel préparé à partir d'a-La purifié ; triangle plein pour l'hydrogel préparé à partir de lactosérum enrichi à 45% d'a-La) et visqueux G" (cercle vide pour l'hydrogel préparé à partir d'a-La purifié ; triangle vide pour l'hydrogel préparé à partir de lactosérum enrichi à 45% d'a-La) en fonction du temps et de la déformation imposée (croix), ont été appliquées ; les contraintes appliquées au cours des étapes 1 à 3 sont détaillées dans l'exemple 2 qui suit.

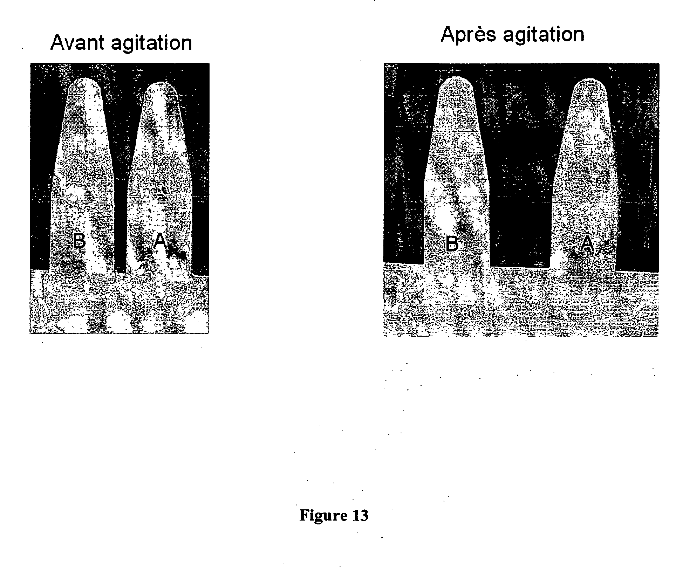

La **Figure 13** comprend deux clichés de tubes Ependorf comprenant, d'une part, un hydrogel réalisé selon le protocole de la thèse de C. Blanchet (B) et, d'autre part, un hydrogel selon l'invention (A). Le cliché de gauche montre ces deux hydrogels après leur préparation (les deux sont dans l'extrémité conique des tubes) ; le cliché de droite montre ces deux hydrogels après agitation, l'hydrogel (A) selon l'invention est dans la partie basse du tube Ependorf alors que l'hydrogel (B) est resté dans la partie haute (extrémité conique).

**Exemple 1 - détermination de la gamme de valeur du nombre de Reynold de l'intensité l'agitation pour la mise en oeuvre du procédé selon l'invention**

Le nombre de Reynolds représente l'intensité de l'agitation ; dans un réacteur agité, il est égal à $Re = \dfrac{\rho V d}{\mu}$

où :

- $\rho$ est la masse volumique du fluide mélangé en Kg.m$^{-3}$,
- $V$ est la vitesse de rotation du barreau magnétique en (m/s),
- $\mu$ est la viscosité du fluide mélangé en (Pa.s) et
- $d$ est la dimension de l'outil d'agitation (par exemple, la longueur pour le cas d'un barreau magnétique) (en m).

La vitesse de rotation N du barreau magnétique est définie par la vitesse imposée par l'agitateur. Pour une gamme de vitesse allant de 0 à 300 rpm.

La relation entre la vitesse V en m/s et la vitesse de rotation N en rpm est la suivante : $V = \dfrac{\pi d}{30} N$

Soit on peut évaluer un nombre de Reynolds : $Re = \dfrac{\rho \pi d^2}{30\mu} N$.

Les fluides mélangés (poudre d'alpha-lactalbumine + suspension aqueuse) ont une viscosité très proche de celle de l'eau car la concentration en poudre d'alpha-lactalbumine est suffisamment faible pour ne pas modifier fortement la viscosité de la suspension lors de son introduction dans l'eau : en conséquence lors du mélange initial de la suspension, la viscosité et la masse volumique de la suspension agitée serra prise égale à celle de l'eau soit : $\rho = 1000$ K/$\mu^3$, $\mu = 10^{-3}$ Pa.s.

La taille du barreau magnétique utilisé est de d = 6x 10$^{-3}$ m de longueur.

Le tableau ci-dessous regroupe des exemples de valeurs de nombres de Reynolds adaptées au mélange préconisé pour obtenir des gels thixotropes à partir de suspensions d'alpha-lactalbumine :

$$Re = \frac{1000.\pi.(6.10^{-3})^2}{30.10^{-3}} N = 1.2\,\pi\,N$$

| N (rpm) | V (m/s) = (2x10$^{-4}\pi$) x N | Re = 1.2 $\pi$ N |
|---|---|---|
| 10 | 2$\pi$ x10$^{-3}$ | 12 $\pi$ = 37.699 |

(suite)

| N (rpm) | V (m/s) = (2x10$^{-4}\pi$) x N | Re = 1.2 $\pi$ N |
|---------|------------------------------|------------------|
| 100 | $2\pi$ x10$^{-2}$ | 120 $\pi$ = 376.99 |
| 300 | $2\pi$ x 0.03 | 360 $\pi$ = 1130.973 |

## Exemple 2 - préparation d'hydrogels d'$\alpha$-lactalbumine selon l'invention

### 2.1. Hydrogel préparé à partir d'a-La purifiée

La protéine purifiée, « $\alpha$-lactalbumin from bovine milk Type III, calcium depleted, $\geq$85% » vendue sous la référence catalogue «L6010» commercialisée par Sigma, et lyophilisée est resuspendue dans une solution aqueuse d'HCl contenant ou non du NaCl.

La concentration d'HCl dépend de la concentration finale d'a-La. Elle est calculée en mM en ajoutant 10 à la valeur numérique de la concentration souhaitée en a-La.

Par exemple, si on souhaite se placer à 40 mg/mL d'a-La, la concentration d'HCl pour la resuspendre sera de 40 + 10 = 50 mM.

Tout d'abord, il faut préparer la solution d'HCl à la concentration déterminée puis y ajouter du NaCl entre 0 et 60 mM. Ensuite, il faut peser la quantité d'a-La nécessaire. Cette quantité dépend de la concentration finale de protéine et du volume de gel à préparer. Les concentrations en a-La utilisées s'étendent de 5 à 60 mg/mL.

La protéine est dissoute dans le volume défini de solution d'HCl puis le pH est ajusté à 2,0$\pm$0,1 avec quelques microlitres d'HCl 1M. La solution est placée sous agitation magnétique à l'aide d'un barreau aimanté et incubée sur la nuit à une température pouvant aller de 37 à 45°C. Le lendemain soit environ 16h plus tard, le gel est formé.

### 2.2. Hydrogel préparé à partir de lactosérum enrichi à 45% d'a-La

Le protocole qui précède est reproduit en utilisant un lactosérum enrichi en a-La 45% en poids fourni par la Société Armor Protéines.

## Exemple 3 - Détermination des caractéristiques viscoélastiques des hydrogels d'a-La par rhéologie

### *Notions de rhéologie*

La rhéologie est une branche de la physique qui étudie l'écoulement ou la déformation des corps sous l'effet des contraintes qui leur sont appliquées, compte tenu de la vitesse d'application de ces contraintes ou plus généralement de leur variation au cours du temps.

A haute concentration en $\alpha$-lactalbumine, la formation de fibres amyloïdes s'accompagne d'une augmentation de viscosité de la solution. Lorsque les fibres sont formées, elles interagissent entre elles pour former un gel. Cette augmentation de viscosité est suivie par rhéométrie. L'échantillon placé dans un rhéomètre va être soumis à une certaine contrainte ($\tau$) dépendante de la vitesse de cisaillement ($\dot{\gamma}$) appliquée. La contraint $\tau$ varie avec le taux de cisaillement $\dot{\gamma}$ et le rapport entre les deux permet de déterminer la viscosité ($\eta$) du fluide étudié. Lorsque $\tau$ est proportionnelle à $\dot{\gamma}$, alors $\eta$ est une constante et le fluide est Newtonien tandis que si $\tau$ n'est pas proportionnelle à $\dot{\gamma}$, alors le fluide est non-newtonien et peut être de différentes natures :

- si la viscosité $\eta$ diminue quand $\tau$ et $\dot{\gamma}$ augmentent, alors on a un fluide rhéofluidifiant ;
- à l'inverse, si $\eta$ augmente quand $\tau$ et $\dot{\gamma}$ augmentent, alors on a un fluide rhéoépaississant.

Les fluides thixotropes sont rhéofluidifiants, leur viscosité diminue sous une même contrainte au cours du temps à cause d'une déstructuration du matériau. Ces fluides sont réversibles puisque lorsque la contrainte est stoppée, le matériau se restructure pour retrouver ses caractéristiques viscoélastiques initiales.

Les caractéristiques viscoélastiques d'un matériau sont obtenues par la détermination des modules dynamiques de viscosité selon la loi de Hooke : $\tau = G\dot{\gamma}$ où G possède deux composantes, G' et G", qui servent à quantifier le comportement visqueux ou élastique des matériaux. G' est le module de stockage (élastique) et G" est le module de perte (visqueux). Quand le caractère élastique domine, G'>>G" et à l'inverse quand le caractère visqueux domine, G'<<G".

**Comportement rhéométrique des gels**

*3.1. Mesures rhéométriques*

La caractérisation du comportement sous écoulement de cisaillement des gels d'a-La a été effectuée en rhéométrie rotative. Les mesures ont été réalisées à l'aide d'un rhéomètre rotatif à couple imposé (ARG2, TA Instrument, 78 Guyancourt, France). Les géométries utilisées sont des géométries cône-plan en titane (angle 4°, diamètre 20 mm, troncature 113 μm). Pour éviter l'évaporation de l'échantillon au cours des mesures, l'atmosphère a été saturée en eau autour de l'échantillon. Pour les mesures en cisaillement harmonique, une étude préliminaire a permis de définir les niveaux de déformation et de fréquence optimum pour lesquelles les mesures appartiennent au domaine de régime linéaire. Dans ce domaine, la sollicitation de cisaillement harmonique imposé ne modifie pas le comportement rhéologique des suspensions, et ne fait que sonder les modules viscoélastiques des gels sans les perturber. La fréquence de 0.1 Hz a été définie comme appartenant au régime linéaire quelle que soit la déformation imposée et le temps de restructuration des échantillons. L'ensemble des mesures en cisaillement harmonique sera donc effectué à cette fréquence de 0.1 Hz. Dans le suivi de la restructuration, une déformation $\gamma$ de 0.01, a aussi été définie comme ne perturbant pas la mesure de G' et G", et sera systématiquement utilisée pour suivre la restructuration des échantillons.

*3.2. Suivi temporel de déstructuration - restructuration des gels sous cisaillement*

3.2.1. Comportement en petites déformations

Une procédure de suivi de consistance par cisaillement harmonique a été mise en place et utilisée de manière systématique pour différents échantillons à des conditions de concentration et force ionique données.
La **Figure 2** présente une succession de cisaillements harmoniques pour un gel d'a-La (20 mg/mL a-La - 30 mM NaCl) suivant les conditions d'amplitude de déformation rapportées dans le Tableau I ci-dessous :

*Tableau I : Conditions de déformation appliquées lors de la procédure de déstructuration-restructuration des gels d'a-La en cisaillement harmonique.*

| Etape | $\dot{\gamma}$ |
|---|---|
| 1- Suivi temporel de la restructuration suite à la mise en place du gel dans l'entrefer des outils. | 0.01 |
| 2- Rampe en déformation pour déstructurer le gel | 0.01 à 10 |
| 3- Suivi temporel de la restructuration | 0.01 |
| 4- Rampe en déformation pour déstructurer le gel | 0.01 à 10 |
| 5- Suivi temporel de la restructuration | 0.01 |

Lors des étapes 1, 3 et 5 à amplitude de déformation constante, on peut mettre en évidence la reprise de consistance du gel correspondant à sa restructuration.
Lors des étapes 2 et 4, l'augmentation progressive de l'amplitude de déformation permet de suivre la déstructuration du gel occasionnée par le cisaillement précédent. A amplitude de déformation croissante, les modules élastique G' et visqueux G" diminuent régulièrement jusqu'à une déformation critique $\gamma_c$ au-delà de laquelle les niveaux chutent fortement ce qui met en évidence la déstructuration du gel et le passage d'un comportement élastique à un comportement visqueux (G' devient inférieur à G").
La reprise de consistance au début des étapes 3 et 5, caractérisée par l'augmentation de G' et G" au cours du temps, met clairement en évidence le comportement thixotrope du gel. Lors de la restructuration, on peut remarquer qu'il existe un temps court Tr1 de restructuration avec un forte reprise de consistance et de l'ordre de 300 s suivi d'un temps plus long Tr2 au cours duquel les augmentations de G' et G" suivent une cinétique plus lente.
Les mêmes observations peuvent être faites en appliquant les trois premières étapes décrites ci-dessus à l'hydrogel préparé à partir de lactosérum enrichi à 45% en poids d'a-La ; le gel obtenu est donc lui aussi thixotrope.

3.2.2. Comportement en grandes déformations

Afin de mettre en évidence le comportement thixotrope sur des déformations de plus grandes amplitudes, un cisaillement simple en grande déformation a été imposé, suivi par un cisaillement harmonique en petites déformations pour suivre la reprise de consistance du gel au cours du temps. Afin de mettre en évidence l'effet du taux de cisaillement sur le niveau de déstructuration atteint ainsi que sur la cinétique de restructuration, différents gradients de cisaillement en grande déformation ont été appliqués.

*Tableau II: Conditions de déformation appliquées lors des procédures de déstructuration en grande déformation et de suivi de restructuration des gels d'a-La en cisaillement harmonique.*

| Etapes | $\dot{\gamma}$ |
|---|---|
| 6 - Suivi temporel de la restructuration suite à une déstructuration en grande déformation à 10 s$^{-1}$ | 0.01 |
| 7 - Rampe en déformation pour déstructurer le gel | 0.01 à 10 |
| 8 - Suivi temporel de la restructuration | 0.01 |

Sur la **Figure 3,** est représentée la reprise de consistance (étape 6) après un cisaillement en grande déformation à un gradient de cisaillement de 10 s$^{-1}$ pendant 300 s. On peut noter à nouveau qu'il existe une première période Tr1 sur laquelle les modules élastiques et visqueux croissent fortement avec le temps, et une deuxième période Tr2 pour laquelle une cinétique de restructuration est beaucoup plus lente.

A la suite d'un long suivi de restructuration sur 1000 min (plus de 16 h), une procédure de déstructuration en cisaillement harmonique à amplitude de déformation croissante est à nouveau appliquée (étape 7) (voir la **Figure 4**), suivie d'une reprise de consistance à petite déformation (étape 8) (voir la **Figure 4**).

Sur la **Figure 5,** sont comparées les deux reprises de consistance soit après un cisaillement simple en grande déformation (étape 6), soit après un cisaillement harmonique à amplitude de déformation croissante (étape 8). Les résultats mettent en évidence des cinétiques de restructuration différentes suivant ces deux modes de déstructuration employés. La cinétique de déformation croissante suite à un cisaillement simple en grande déformation est beaucoup plus lente que lors d'un cisaillement harmonique en petite déformation à amplitude croissante. En effet le cisaillement en grande déformation parvient à déstructurer l'échantillon à un niveau supérieur que celui obtenu lors d'un cisaillement en petite déformation. Ce résultat met à nouveau en évidence l'importance du type de sollicitation et de son intensité sur le niveau de déstructuration atteint dans l'échantillon lors de son cisaillement, ce qui est révélateur du comportement de systèmes thixotropes.

Sur la **Figure 6,** est représentée l'évolution des modules viscoélastiques en fonction de la déformation, mesurés lors d'une procédure de déstructuration (étape 7) en cisaillement harmonique à amplitude de déformation croissante. Il est mis en évidence que la déformation critique $\gamma_c$ au-delà de laquelle le gel commence à couler, identifié par le croisement de G' et G", est de l'ordre de 0.2.

### 3.3. Effet de la force ionique sur le comportement thixotrope des gels d'a-La

Afin d'évaluer les différences de cinétique de restructuration-déstructuration des gels d'a-La ainsi que des niveaux de consistance atteints en fonction de la force ionique en NaCl, une procédure identique à celle présentée dans le Tableau I, a été mise en oeuvre sur diverses suspensions d'a-La. Les résultats sont présentés dans les **Figures 7** à **9.**

Sur la **Figure 10,** est représentée l'évolution des modules viscoélastiques G' et G" en fonction de la force ionique en NaCl et de la concentration en a-La. Les niveaux ont été comparés au même temps de restructuration 500 s de l'étape 3 correspondant à la zone de cinétique lente de restructuration, c'est à dire sur le « plateau » atteint lors de la restructuration. Il est mis en évidence que les niveaux de G' et G" diminuent lorsque la force ionique augmente, ce qui correspond à une réduction de la consistance du gel. L'augmentation de la concentration en a-La entraîne une augmentation des modules viscoélastiques.

Sur la **Figure 11,** sont comparées les déstructurations appliquées sur divers échantillons ; ces résultats mettent en évidence que la déformation critique se déplace vers des niveaux plus élevés lorsque la force ionique augmente ou lorsque la concentration en protéine diminue. Des déstructurations successives d'un même échantillon présentent des déformations critiques du même ordre de grandeur (non présenté sur la figure) ce qui met en évidence la très bonne stabilité du système à subir des sollicitations successives de déstructuration-restructuration, ainsi qu'une très bonne stabilité dans le temps de par la reproductibilité des mesures en G' et G".

### Exemple 4 - préparation d'un hydrogel d'$\alpha$-lactalbumine selon les conditions décrites dans la thèse de C. Blanchet

Le but de cet essai et de reproduire une suspension d'a-La susceptible d'être obtenue par le protocole décrit par C. *Blanchet et al.* puis de caractériser ses propriétés rhéologiques.

Les expériences ont été conduites dans les conditions et selon le protocole décrits à la page 203 de la thèse : les protéines d'a-La (10 mg/mL avec 30 mM de NaCl) sont mises en suspension, ensuite, le pH de cette suspension est ajusté à 2 puis la suspension est introduite dans un tube Ependorf ; le tube est agité à 40°C (les conditions d'agitation sont celles utilisées pour la préparation des hydrogels selon l'invention).

En parallèle, un hydrogel à 10 mg/mL d'a-La avec 30 mM de NaCl est préparé par le procédé selon l'invention.

Le cliché de gauche de la **Figure 13** illustre l'aspect des hydrogels ainsi obtenus ((B) selon la thèse et (A) selon l'invention) : l'hydrogel (B) présente un aspect moins homogène que l'hydrogel (A).

On observe également que ces deux hydrogels n'ont pas le même comportement lorsqu'on les agite : le cliché de droite de la **Figure 13** montre ces deux hydrogels après agitation. En raison de son comportement thixotrope, la viscosité de l'hydrogel (A) selon l'invention a diminué lors de l'agitation et il a coulé dans la partie basse du tube Ependorf ; *a contrario,* l'agitation n'a pas provoqué d'écoulement de l'hydrogel (B) qui est resté dans la partie haute du tube Ependorf (extrémité conique).

## Exemple 5 - préparation d'un hydrogel d'α-lactalbumine à pH 7 et 80°C

Le but de cet essai et de reproduire l'hydrogel décrit par Kavanagh, G. M., A. H. Clark, et al. (2000). "Heat-induced gelation of beta-lactoglobulin/ alpha-lactalbumin blends at pH 3 and pH 7." Macromolecules 33(19): 7029-7037*,* puis de caractériser ses propriétés rhéologiques.

Conditions décrites dans l'article :

Concentration en a-La de 15% (w/w), c'est-à-dire 150 mg/mL
T°C = 80°C
Solvant = eau déionisée
pH = 7.0
Les gels sont observés au bout de 1 à 2h à 80°C.

Conditions mises en oeuvre :

Concentration en a-La de 15% (w/w), c'est-à-dire 150 mg/mL
T°C = 80°C pendant 1h
Solvant = eau déionisée
pH = 7.2

La vérification de la concentration en a-La est réalisée en mesurant l'absorbance à 280 nm de la solution à l'aide d'un spectrophotomètre Nanodrop® ND-1000 (LabTech) : les mesures sont réalisées sur trois solutions de protéines diluées chacune au cinquième : A1 = 61 ; A2 = 62 ; A3 = 61. La concentration d'a-La est déterminée selon la loi de Beer-Lambert $A = \varepsilon C \, 1$ où $\varepsilon$ = 27880 L·mor$^{-1}$·cm$^{-1}$ et 1 = 1 cm. La concentration molaire C de la solution d'a-La est donc de 10.9 mM ce qui correspond à une concentration massique de 154 mg/mL (la masse molaire de l'a-La est de 14150 g mol$^{-1}$).

La solution d'a-La préparée est séparée en deux tubes de 200 $\mu$L. Un tube est placé à 80°C, 1h, sans agitation et l'autre tube est conservé à température ambiante comme témoin.

En moins d'une heure, un hydrogel s'est formé dans le tube placé à 80°C. Le gel obtenu est dur, un cône ne s'enfonce pas dedans mais montre une certaine élasticité. Il peut également être démoulé en conservant la forme du tube ce qui n'est pas le cas des hydrogels selon l'invention qui présentent une consistance plus molle.

L'hydrogel ainsi obtenu présente également un aspect plus transparent alors que les hydrogels selon l'invention sont translucides (ils laissent passer une lumière diffuse mais on ne peut pas distinguer des objets au travers de ces hydrogels). Enfin, si on le secoue fortement, il ne change pas de forme, il est irréversible.

L'hydrogel produit ici ne présente ainsi ni l'aspect ni les propriétés physiques des gels thixotropes réalisés selon le procédé de l'invention.

BIBLIOGRAPHIE

Akkermans, C., P. Venema, et al. (2008). "Peptides are building blocks of heat-induced fibrillar protein aggregates of beta-lactoglobulin formed at pH 2." Biomacromolecules 9(5): 1474-1479.
Audic, J. L., B. Chaufer, et al. (2003). "Non-food applications of milk components and dairy co-products: A review."

Lait 83(6): 417-438.

Chen, H. (1995). "Functional properties and applications of edible films made of milk proteins." Journal of Dairy Science 78(11): 2563-2583.

Gosal, W. S., A. H. Clark, et al. (2004). "Fibrillar beta-lactoglobulin gels: Part 1. Fibril formation and structure." Biomacromolecules 5(6): 2408-2419.

Gosal, W. S., A. H. Clark, et al. (2004). "Fibrillar beta-lactoglobulin gels: Part 2. Dynamic mechanical characterization of heat-set systems." Biomacromolecules 5(6): 2420-2429.

Gosal, W. S., A. H. Clark, et al. (2004). "Fibrillar beta-lactoglobulin gels: Part 3. Dynamic mechanical of solvent-induced systems." Biomacromolecules 5(6): 2430-2438.

Graveland-Bikker, J. F., R. Ipsen, et al. (2004). "Influence of calcium on the self-assembly of partially hydrolyzed alpha-lactalbumin." Langmuir 20(16): 6841-6846.

Gunasekaran, S., S. Ko, et al. (2007). "Use of whey proteins for encapsulation and controlled delivery applications." Journal of Food Engineering 83(1): 31-40.

Heidebach, T., P. Forst, et al. (2009). "Microencapsulation of probiotic cells by means of rennet-gelation of milk proteins." FOOD HYDROCOLLOIDS 23(7): 1670-1677.

Heidebach, T., P. Forst, et al. (2009). "Transglutaminase-induced caseinate gelation for the microencapsulation of probiotic cells." INTERNATIONAL DAIRY JOURNAL 19(2): 77-84.

Hines, M. E. and E. A. Foegeding (1993). "INTERACTIONS OF ALPHA-LACTALBUMIN AND BOVINE SERUM-ALBUMIN WITH BETA-LACTOGLOBULIN IN THERMALLY INDUCED GELATION." Journal of Agricultural and Food Chemistry 41(3): 341-346.

Holmes, T. C., S. de Lacalle, et al. (2000). "Extensive neurite outgrowth and active synapse formation on self-assembling peptide scaffolds." Proceedings of the National Academy of Sciences of the United States of America 97(12): 6728-6733.

Ipsen, R. and J. Otte (2007). "Self-assembly of partially hydrolysed alpha-lactalbumin." BIOTECHNOLOGY ADVANCES 25(6): 602-605.

Ipsen, R., J. Otte, et al. (2001). "Molecular self-assembly of partially hydrolysed alpha-lactalbumin resulting in strong gels with a novel microstructure." Journal of Dairy Research 68(2): 277-286.

Kamau, S. M., S. C. Cheison, et al. (2010). "Alpha-Lactalbumin: Its Production Technologies and Bioactive Peptides." Comprehensive Reviews in Food Science and Food Safety 9(2): 197-212.

Kavanagh, G. M., A. H. Clark, et al. (2000). "Heat-induced gelation of beta-lactoglobulin/alpha-lactalbumin blends at pH 3 and pH 7." Macromolecules 33(19): 7029-7037.

Kavanagh, G. M., A. H. Clark, et al. (2000). "Heat-induced gelation of globular proteins: part 3. Molecular studies on low pH beta-lactoglobulin gels." International Journal of Biological Macromolecules 28(1): 41-50.

Kopecek, J. and J. Yang (2009). "Peptide-directed self-assembly of hydrogels." Acta Biomater 5(3): 805-816.

Kyle, S., A. Aggeli, et al. (2009). "Production of self-assembling biomaterials for tissue engineering." Trends in Biotechnology 27(7): 423-433.

Le Tien, C., C. Vachon, et al. (2001). "Milk protein coatings prevent oxidative browning of apples and potatoes." Journal of Food Science 66(4): 512-516.

Livney, Y. D. (2010). "Milk proteins as vehicles for bioactives." Current Opinion in Colloid & Interface Science 15(1-2): 73-83.

Loveday, S. M., M. A. Rao, et al. (2009). "Factors Affecting Rheological Characteristics of Fibril Gels: The Case of beta-Lactoglobulin and alpha-Lactalbumin." Journal of Food Science 74(3): R47-R55.

Madureira, A. R., C. I. Pereira, et al. (2007). "Bovine whey proteins - Overview on their main biological properties." Food Research International 40(10): 1197-1211.

Mewis, J. (1979). "THIXOTROPY - GENERAL-REVIEW." Journal of Non-Newtonian Fluid Mechanics 6(1): 1-20.

Oboroceanu, D., L. Wang, et al. (2010). "Characterization of $\beta$-Lactoglobulin Fibrillar Assembly Using Atomic Force Microscopy, Polyacrylamide Gel Electrophoresis, and in Situ Fourier Transform Infrared Spectroscopy." Journal of Agricultural and Food Chemistry.

Paulsson, M., P. O. Hegg, et al. (1986). "HEAT-INDUCED GELATION OF INDIVIDUAL WHEY PROTEINS A DYNAMIC RHEOLOGICAL STUDY." Journal of Food Science 51(1): 87-90.

Pek, Y. S., A. C. A. Wan, et al. (2008). "A thixotropic nanocomposite gel for three-dimensional cell culture." Nature Nanotechnology 3(11): 671-675.

Permyakov, E. A. and L. J. Berliner (2000). "alpha-Lactalbumin: structure and function." Febs Letters 473(3): 269-274.

Piau, J. M. (2007). "Carbopol gels: Elastoviscoplastic and slippery glasses made of individual swollen sponges Meso- and macroscopic properties, constitutive équations and scaling laws." Journal of Non-Newtonian Fluid Mechanics 144(1): 1-29.

Pignon, F., A. Magnin, et al. (1998). "Thixotropic behavior of clay dispersions: Combinations of scattering and rheometric techniques." Journal of Rheology 42(6): 1349-1373.

Restani, P., C. Ballabio, et al. (2009). "Molecular aspects of milk allergens and their role in clinical events." ANALYTICAL AND BIOANALYTICAL CHEMISTRY 395(1): 47-56.

Rodrigues, M. M. A., A. R. Simioni, et al. (2009). "Préparation, characterization and in vitro cytotoxicity of BSA-based nanospheres containing nanosized magnetic particles and/or photosensitizer." Journal of Magnetism and Magnetic Materials 321(10): 1600-1603.

Semo, E., E. Kesselman, et al. (2007). "Casein micelle as a natural nano-capsular vehicle for nutraceuticals." Food Hydrocolloids 21(5-6): 936-942.

Song, F., L. Zhang, et al. (2009). "Genipin-crosslinked casein hydrogels for controlled drug delivery." INTERNATIONAL JOURNAL OF PHARMACEUTICS 373(1-2): 41-47.

Tokpavi, D. L., P. Jay, et al. (2009). "Experimental study of the very slow flow of a yield stress fluid around a circular cylinder." Journal of Non-Newtonian Fluid Mechanics 164(1-3): 35-44.

van der Linden, E. and P. Venema (2007). "Self-assembly and aggregation of proteins." Current Opinion in Colloid & Interface Science 12(4-5): 158-165.

Vintiloiu, A. and J.-C. Leroux (2008). "Organogels and their use in drug delivery -- A review." JOURNAL OF CONTROLLED RELEASE 125(3): 179-192.

Yan, H., H. Frielinghaus, et al. (2008). "Thermoreversible lysozyme hydrogels: properties and an insight into the gelation pathway." Soft Matter 4(6): 1313-1325.

Yan, H., A. Saiani, et al. (2006). "Thermoreversible Protein Hydrogel as Cell Scaffold." Biomacromolecules 7(10): 2776-2782.

Yanlian, Y., K. Ulung, et al. (2009). "Designer self-assembling peptide nanomaterials." Nano Today 4(2): 193-210.

Zhang, S. (2008). Designer self-assembling peptide nanofiber scaffolds for study of 3-D cell biology and beyond. Advances in Cancer Research, Vol 99. San Diego, Elsevier Academic Press Inc. 99: 335-+.

## Revendications

1. Procédé de préparation d'un hydrogel d'α-lactalbumine à partir d'une suspension aqueuse d'α-lactalbumine à une concentration $C_{a-La}$ comprise entre 5 et 60 mg/mL, comprenant les étapes suivantes :

   a) la mise en suspension de l'α-lactalbumine dans une solution aqueuse acide ayant une force ionique inférieure ou égale à 60 mM ; ladite mise en suspension consistant en :

   (a1) la préparation d'une solution aqueuse acide ayant une concentration en protons exprimée en mM déterminée par la somme : (valeur numérique de $C_{a-La}$ exprimée en g/L) + 10 ;
   (a2) la mise en suspension de l'α-lactalbumine dans ladite solution aqueuse acide ; et
   (a3) si nécessaire, l'ajustement du pH à une valeur comprise entre 1,5 et 2,5 ;

   b) la formation du gel à partir de ladite suspension d'α-lactalbumine obtenue à l'issue de l'étape a) ; ladite formation du gel est mise en oeuvre dans les conditions suivantes :

   - à une température inférieure à 60°C ;
   - sous une agitation ayant une intensité définie par un nombre de Reynolds compris entre 37 et 1000 ;
   - pendant 10 heures à 1 semaine, et
   - en l'absence d'évaporation d'eau de ladite suspension d'α-lactalbumine.

2. Procédé selon la revendication 1, **caractérisé en ce que** le nombre de Reynolds est compris entre 300 et 500.

3. Procédé selon la revendication 1 ou la revendication 2, **caractérisé en ce que** la température est comprise entre 35 et 55°C.

4. Procédé selon l'une quelconque des revendications précédentes, **caractérisé en ce que** le pH est compris entre 1,8 et 2,2.

5. Procédé selon l'une quelconque des revendications précédentes, **caractérisé en ce que** ladite suspension d'α-lactalbumine obtenue à l'issue de l'étape a) est filtrée.

6. Hydrogel rhéofluidifiant, à seuil et thixotrope d'α-lactalbumine susceptible d'être obtenu selon le procédé des revendications 1 à 5.

**7.** Hydrogel rhéofluidifiant, à seuil et thixotrope d'α-lactalbumine ayant une teneur en α-lactalbumine comprise entre 5 et 60 mg/mL, un pH compris entre 1,5 et 2,5 et une force ionique inférieure ou égale à 60 mM.

**8.** Utilisation d'un hydrogel rhéofluidifiant, à seuil et thixotrope d'α-lactalbumine ayant une teneur en α-lactalbumine comprise entre 5 et 60 mg/mL, comme agent texturant alimentaire.

**9.** Produit alimentaire comprenant au moins un hydrogel rhéofluidifiant, à seuil et thixotrope d'α-lactalbumine ayant une teneur en α-lactalbumine comprise entre 5 et 60 mg/mL.

**10.** Hydrogel rhéofluidifiant, à seuil et thixotrope d'α-lactalbumine ayant une teneur en α-lactalbumine comprise entre 5 et 60 mg/mL, pour une utilisation pour le traitement et/ou la cicatrisation des plaies.

**11.** Pansement comprenant au moins un hydrogel rhéofluidifiant, à seuil et thixotrope d'α-lactalbumine ayant une teneur en α-lactalbumine comprise entre 5 et 60 mg/mL, et, optionnellement au moins un composé actif tel qu'un agent cicatrisant ou un agent antimicrobien.

**12.** Utilisation d'un hydrogel rhéofluidifiant, à seuil et thixotrope d'α-lactalbumine ayant une teneur en α-lactalbumine comprise entre 5 et 60 mg/mL pour la préparation d'une composition cosmétique.

**13.** Composition cosmétique comprenant au moins un hydrogel rhéofluidifiant, à seuil et thixotrope d'α-lactalbumine ayant une teneur en α-lactalbumine comprise entre 5 et 60 mg/mL.

**14.** Utilisation d'un hydrogel rhéofluidifiant, à seuil et thixotrope d'α-lactalbumine ayant une teneur en α-lactalbumine comprise entre 5 et 60 mg/mL, pour la préparation de peintures.

**15.** Peinture comprenant au moins un hydrogel rhéofluidifiant, à seuil et thixotrope d'α-lactalbumine ayant une teneur en α-lactalbumine comprise entre 5 et 60 mg/mL.

**Patentansprüche**

**1.** Verfahren zur Herstellung eines α-Laktalbumin-Hydrogels aus einer wässrigen Suspension von α-Laktalbumin mit einer Konzentration $C_{a\text{-}La}$ zwischen 5 und 60 mg/ml, umfassend die folgenden Schritte:

a) Suspendieren von α-Laktalbumin in einer sauren wässrigen Lösung, die eine Ionenstärke kleiner oder gleich 60 mM hat; wobei das Suspendieren besteht aus:

(a1) Herstellen einer sauren wässrigen Lösung mit einer in mM ausgedrückten Protonenkonzentration, die bestimmt ist durch die Summe von: (Zahlenwert von $C_{a\text{-}La}$, ausgedrückt in g/l) + 10;
(a2) Suspendieren von α-Laktalbumin in der sauren wässrigen Lösung; und
(a3) falls erforderlich, Einstellen des pHs auf einen Wert zwischen 1,5 und 2,5;

b) Bilden des Gels aus der am Ende von Schritt a) erhaltenen Suspension von α-Laktalbumin; wobei die Bildung des Gels unter den folgenden Bedingungen durchgeführt wird:

- bei einer Temperatur unter 60 °C;
- unter Rühren mit einer Intensität, die durch eine Reynoldszahl zwischen 37 und 1000 definiert ist;
- für 10 Stunden bis 1 Woche, und
- ohne Wasserverdampfung aus der Suspension von α-Laktalbumin.

**2.** Verfahren nach Anspruch 1, **dadurch gekennzeichnet, dass** die Reynoldszahl zwischen 300 und 500 liegt.

**3.** Verfahren nach Anspruch 1 oder Anspruch 2, **dadurch gekennzeichnet, dass** die Temperatur zwischen 35 und 55 °C liegt.

**4.** Verfahren nach einem der vorherigen Ansprüche, **dadurch gekennzeichnet, dass** der pH zwischen 1,8 und 2,2 liegt.

**5.** Verfahren nach einem der vorherigen Ansprüche, **dadurch gekennzeichnet, dass** die am Ende von Schritt a) erhaltene Suspension von $\alpha$-Laktalbumin filtriert wird.

**6.** Strukturviskoses $\alpha$-Laktalbumin-Hydrogel, das eine Fließgrenze hat und thixotrop ist, erhältlich nach dem Verfahren der Ansprüche 1-5.

**7.** Strukturviskoses $\alpha$-Laktalbumin-Hydrogel, das eine Fließgrenze hat und thixotrop ist, mit einem Gehalt an $\alpha$-Laktalbumin zwischen 5 und 60 mg/ml, einem pH zwischen 1,5 und 2,5 und einer Ionenstärke kleiner oder gleich 60 mM.

**8.** Verwendung eines strukturviskosen $\alpha$-Laktalbumin-Hydrogels, das eine Fließgrenze hat und thixotrop ist, mit einem Gehalt an $\alpha$-Laktalbumin zwischen 5 und 60 mg/ml als Lebensmittel-Texturierungsmittel.

**9.** Lebensmittel, umfassend mindestens ein strukturviskoses $\alpha$-Laktalbumin-Hydrogel, das eine Fließgrenze hat und thixotrop ist, mit einem Gehalt an $\alpha$-Laktalbumin zwischen 5 und 60 mg/ml.

**10.** Strukturviskoses $\alpha$-Laktalbumin-Hydrogel, das eine Fließgrenze hat und thixotrop ist, mit einem Gehalt an $\alpha$-Laktalbumin zwischen 5 und 60 mg/ml zur Verwendung für die Behandlung und/oder Heilung von Wunden.

**11.** Verband, umfassend mindestens ein strukturviskoses $\alpha$-Laktalbumin-Hydrogel, das eine Fließgrenze hat und thixotrop ist, mit einem Gehalt an $\alpha$-Laktalbumin zwischen 5 und 60 mg/ml und wahlweise mindestens einen Wirkstoff wie ein Heilmittel oder ein antimikrobielles Mittel.

**12.** Verwendung eines strukturviskosen $\alpha$-Laktalbumin-Hydrogels, das eine Fließgrenze hat und thixotrop ist, mit einem Gehalt an $\alpha$-Laktalbumin zwischen 5 und 60 mg/ml zur Herstellung einer kosmetischen Zusammensetzung.

**13.** Kosmetische Zusammensetzung, umfassend mindestens ein strukturviskoses $\alpha$-Laktalbumin-Hydrogel, das eine Fließgrenze hat und thixotrop ist, mit einem Gehalt an $\alpha$-Laktalbumin zwischen 5 und 60 mg/ml.

**14.** Verwendung eines strukturviskosen $\alpha$-Laktalbumin-Hydrogels, das eine Fließgrenze hat und thixotrop ist, mit einem Gehalt an $\alpha$-Laktalbumin zwischen 5 und 60 mg/ml zur Herstellung von Farben.

**15.** Farbe, umfassend mindestens ein strukturviskoses $\alpha$-Laktalbumin-Hydrogel, das eine Fließgrenze hat und thixotrop ist, mit einem Gehalt an $\alpha$-Laktalbumin zwischen 5 und 60 mg/ml.

**Claims**

**1.** A process for preparing an $\alpha$-lactalbumin hydrogel from an aqueous suspension of $\alpha$-lactalbumin at a concentration $C_{a-La}$ of between 5 and 60 mg/ml, comprising the following steps:

a) suspending the $\alpha$-lactalbumin in an acidic aqueous solution having an ionic strength of less than or equal to 60 mM; said suspending consisting in:

(a1) preparing an acidic aqueous solution having a concentration of protons expressed in mM determined by the sum: (numerical value of $C_{a-La}$ expressed in g/l) + 10;
(a2) suspending the $\alpha$-lactalbumin in said acidic aqueous solution; and
(a3) if necessary, adjusting the pH to a value of between 1.5 and 2.5;

b) forming the gel from said $\alpha$-lactalbumin suspension obtained at the end of step a); said forming of the gel is carried out under the following conditions;

- at a temperature below 60°C;
- with stirring having a strength defined by a Reynolds number of between 37 and 1000;
- for 10 hours to 1 week, and
- in the absence of evaporation of water from said $\alpha$-lactalbumin suspension.

**2.** The process as claimed in claim 1, **characterized in that** the Reynolds number is between 300 and 500.

3. The process as claimed in claim 1 or claim 2, **characterized in that** the temperature is between 35 and 55°C.

4. The process as claimed in any one of the preceding claims, **characterized in that** the pH is between 1.8 and 2.2.

5. The process as claimed in any one of the preceding claims, **characterized in that** said α-lactalbumin suspension obtained at the end of step a) is filtered.

6. An α-lactalbumin shear-thinning hydrogel which has a yield point and is thixotropic, which can be obtained according to the process of claims 1 to 5.

7. An α-lactalbumin shear-thinning hydrogel which has a yield point and is thixotropic, having an α-lactalbumin content of between 5 and 60 mg/ml, a pH of between 1.5 and 2.5 and an ionic strength of less than or equal to 60 mM.

8. The use of an α-lactalbumin shear-thinning hydrogel which has a yield point and is thixotropic, having an α-lactalbumin content of between 5 and 60 mg/ml, as a food texturing agent.

9. A food product comprising at least one α-lactalbumin shear-thinning hydrogel which has a yield point and is thixotropic, having an α-lactalbumin content of between 5 and 60 mg/ml.

10. An α-lactalbumin shear-thinning hydrogel which has a yield point and is thixotropic, having an α-lactalbumin content of between 5 and 60 mg/ml, for use in the treatment and/or healing of wounds.

11. A dressing comprising at least one α-lactalbumin shear-thinning hydrogel which has a yield point and is thixotropic, having an α-lactalbumin content of between 5 and 60 mg/ml, and optionally at least one active compound, such as a healing agent or an antimicrobial agent.

12. The use of an α-lactalbumin shear-thinning hydrogel which has a yield point and is thixotropic, having an α-lactalbumin content of between 5 and 60 mg/ml, for preparing a cosmetic composition.

13. A cosmetic composition comprising at least one α-lactalbumin shear-thinning hydrogel which has a yield point and is thixotropic, having an α-lactalbumin content of between 5 and 60 mg/ml.

14. The use of an α-lactalbumin shear-thinning hydrogel which has a yield point and is thixotropic, having an α-lactalbumin content of between 5 and 60 mg/ml, for preparing paints.

15. A paint comprising at least one α-lactalbumin shear-thinning hydrogel which has a yield point and is thixotropic, having an α-lactalbumin content of between 5 and 60 mg/ml.

**Figure 1**

**Figure 2**

**Alpha-lactalbumine 20 mg/mL - 30 mM NaCl - T = 15°C**

**Figure 3**

**Alpha-lactalbumine 20 mg/mL - 30 mM NaCl - T = 15°C**

**Figure 4**

**Alpha-lactalbumine 20 mg/mL - 30 mM NaCl - T = 15°C**

**Figure 5**

**Alpha-lactalbumine 20 mg/mL - 30 mM NaCl - T = 15°C**

**Figure 6**

Alpha-lactalbumine 20 mg/mL - 0 mM NaCl - T = 15 °C

**Figure 7**

Alpha-lactalbumine 20 mg/mL - 60 mM NaCl - T = 15 °C

**Figure 8**

**Alpha-lactalbumine 40 mg/mL - 60 mM NaCl - T = 15 °C**

**Figure 9**

**Figure 10**

**Figure 11**

Alphalac 20 mg/mL - 0 mM NaCl - T = 15 °C

**Figure 12**

Avant agitation

Après agitation

Figure 13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008130252 A

**Littérature non-brevet citée dans la description**

- **C. BLANCHET.** *Repliement des protéines et formation de fibres amyloïdes. Le cas de l'$\alpha$-lactalbumine,* 23 Juin 2008
- **KAVANAGH, G. M. ; A. H. CLARK et al.** Heat-induced gelation of beta-lactoglobulin/ alpha-lactalbumin blends at pH 3 and pH 7. *Macromolecules,* 2000, vol. 33 (19), 7029-7037
- **AKKERMANS, C. ; P. VENEMA et al.** Peptides are building blocks of heat-induced fibrillar protein aggregates of beta-lactoglobulin formed at pH 2. *Biomacromolecules,* 2008, vol. 9 (5), 1474-1479
- **AUDIC, J. L. ; B. CHAUFER et al.** Non-food applications of milk components and dairy co-products: A review. *Lait,* 2003, vol. 83 (6), 417-438
- **CHEN, H.** Functional properties and applications of edible films made of milk proteins. *Journal of Dairy Science,* 1995, vol. 78 (11), 2563-2583
- **GOSAL, W. S. ; A. H. CLARK et al.** Fibrillar beta-lactoglobulin gels: Part 1. Fibril formation and structure. *Biomacromolecules,* 2004, vol. 5 (6), 2408-2419
- **GOSAL, W. S. ; A. H. CLARK et al.** Fibrillar beta-lactoglobulin gels: Part 2. Dynamic mechanical characterization of heat-set systems. *Biomacromolecules,* 2004, vol. 5 (6), 2420-2429
- **GOSAL, W. S. ; A. H. CLARK et al.** Fibrillar beta-lactoglobulin gels: Part 3. Dynamic mechanical of solvent-induced systems. *Biomacromolecules,* 2004, vol. 5 (6), 2430-2438
- **GRAVELAND-BIKKER, J. F. ; R. IPSEN et al.** Influence of calcium on the self-assembly of partially hydrolyzed alpha-lactaibumin. *Langmuir,* 2004, vol. 20 (16), 6841-6846
- **GUNASEKARAN, S. ; S. KO et al.** Use of whey proteins for encapsulation and controlled delivery applications. *Journal of Food Engineering,* 2007, vol. 83 (1), 31-40
- **HEIDEBACH, T. ; P. FORST et al.** Microencapsulation of probiotic cells by means of rennet-gelation of milk proteins. *FOOD HYDROCOLLOIDS,* 2009, vol. 23 (7), 1670-1677

- **HEIDEBACH, T. ; P. FORST et al.** Transglutaminase-induced caseinate gelation for the microencapsulation of probiotic cells. *INTERNATIONAL DAIRY JOURNAL,* 2009, vol. 19 (2), 77-84
- **HINES, M. E. ; E. A. FOEGEDING.** INTERACTIONS OF ALPHA-LACTALBUMIN AND BOVINE SERUM-ALBUMIN WITH BETA-LACTOGLOBULIN IN THERMALLY INDUCED GELATION. *Journal of Agricultural and Food Chemistry,* 1993, vol. 41 (3), 341-346
- **HOLMES, T. C. ; S. DE LACALLE et al.** Extensive neurite outgrowth and active synapse formation on self-assembling peptide scaffolds. *Proceedings of the National Academy of Sciences of the United States of America,* 2000, vol. 97 (12), 6728-6733
- **IPSEN, R. ; J. OTTE.** Self-assembly of partially hydrolysed alpha-lactalbumin. *BIOTECHNOLOGY ADVANCES,* 2007, vol. 25 (6), 602-605
- **IPSEN, R. ; J. OTTE et al.** Molecular self-assembly of partially hydrolysed alpha-lactalbumin resulting in strong gels with a novel microstructure. *Journal of Dairy Research,* 2001, vol. 68 (2), 277-286
- **KAMAU, S. M. ; S. C. CHEISON et al.** Alpha-Lactalbumin: Its Production Technologies and Bioactive Peptides. *Comprehensive Reviews in Food Science and Food Safety,* 2010, vol. 9 (2), 197-212
- **KAVANAGH, G. M. ; A. H. CLARK et al.** Heat-induced gelation of beta-lactoglobulin/alpha-lactalbumin blends at pH 3 and pH 7. *Macromolecules,* 2000, vol. 33 (19), 7029-7037
- **KAVANAGH, G. M. ; A. H. CLARK et al.** Heat-induced gelation of globular proteins: part 3. Molecular studies on low pH beta-lactoglobulin gels. *International Journal of Biological Macromolecules,* 2000, vol. 28 (1), 41-50
- **KOPECEK, J. ; J. YANG.** Peptide-directed self-assembly of hydrogels. *Acta Biomater,* 2009, vol. 5 (3), 805-816
- **KYLE, S. ; A. AGGELI et al.** Production of self-assembling biomaterials for tissue engineering. *Trends in Biotechnology,* 2009, vol. 27 (7), 423-433

- **LE TIEN, C. ; C. VACHON et al.** Milk protein coatings prevent oxidative browning of apples and potatoes. *Journal of Food Science,* 2001, vol. 66 (4), 512-516
- **LIVNEY, Y. D.** Milk proteins as vehicles for bioactives. *Current Opinion in Colloid & Interface Science,* 2010, vol. 15 (1-2), 73-83
- **LOVEDAY, S. M. ; M. A. RAO et al.** Factors Affecting Rheological Characteristics of Fibril Gels: The Case of beta-Lactoglobulin and alpha-Lactalbumin. *Journal of Food Science,* 2009, vol. 74 (3), R47-R55
- **MADUREIRA, A. R. ; C. I. PEREIRA et al.** Bovine whey proteins - Overview on their main biological properties. *Food Research International,* 2007, vol. 40 (10), 1197-1211
- **MEWIS, J.** THIXOTROPY - GENERAL-REVIEW. *Journal of Non-Newtonian Fluid Mechanics,* 1979, vol. 6 (1), 1-20
- **OBOROCEANU, D. ; L. WANG et al.** Characterization of β-Lactoglobulin Fibrillar Assembly Using Atomic Force Microscopy, Polyacrylamide Gel Electrophoresis, and in Situ Fourier Transform Infrared Spectroscopy. *Journal of Agricultural and Food Chemistry,* 2010
- **PAULSSON, M. ; P. O. HEGG et al.** HEAT-INDUCED GELATION OF INDIVIDUAL WHEY PROTEINS A DYNAMIC RHEOLOGICAL STUDY. *Journal of Food Science,* 1986, vol. 51 (1), 87-90
- **PEK, Y. S. ; A. C. A. WAN et al.** A thixotropic nanocomposite gel for three-dimensional cell culture. *Nature Nanotechnology,* 2008, vol. 3 (11), 671-675
- **PERMYAKOV, E. A. ; L. J. BERLINER.** alpha-Lactalbumin: structure and function. *Febs Letters,* 2000, vol. 473 (3), 269-274
- **PIAU, J. M.** Carbopol gels: Elastoviscoplastic and slippery glasses made of individual swollen sponges Meso- and macroscopic properties, constitutive équations and scaling laws. *Journal of Non-Newtonian Fluid Mechanics,* 2007, vol. 144 (1), 1-29
- **PIGNON, F. ; A. MAGNIN et al.** Thixotropic behavior of clay dispersions: Combinations of scattering and rheometric techniques. *Journal of Rheology,* 1998, vol. 42 (6), 1349-1373
- **RESTANI, P. ; C. BALLABIO et al.** Molecular aspects of milk allergens and their role in clinical events. *ANALYTICAL AND BIOANALYTICAL CHEMISTRY,* 2009, vol. 395 (1), 47-56
- **RODRIGUES, M. M. A. ; A. R. SIMIONI et al.** Préparation, characterization and in vitro cytotoxicity of BSA-based nanospheres containing nanosized magnetic particles and/or photosensitizer. *Journal of Magnetism and Magnetic Materials,* 2009, vol. 321 (10), 1600-1603
- **SEMO, E. ; E. KESSELMAN et al.** Casein micelle as a natural nano-capsular vehicle for nutraceuticals. *Food Hydrocolloids,* 2007, vol. 21 (5-6), 936-942
- **SONG, F. ; L. ZHANG et al.** Genipin-crosslinked casein hydrogels for controlled drug delivery. *INTERNATIONAL JOURNAL OF PHARMACEUTICS,* 2009, vol. 373 (1-2), 41-47
- **TOKPAVI, D. L. ; P. JAY et al.** Experimental study of the very slow flow of a yield stress fluid around a circular cylinder. *Journal of Non-Newtonian Fluid Mechanics,* 2009, vol. 164 (1-3), 35-44
- **VAN DER LINDEN, E. ; P. VENEMA.** Self-assembly and aggregation of proteins. *Current Opinion in Colloid & Interface Science,* 2007, vol. 12 (4-5), 158-165
- **VINTILOIU, A. ; J.-C. LEROUX.** Organogels and their use in drug delivery -- A review. *JOURNAL OF CONTROLLED RELEASE,* 2008, vol. 125 (3), 179-192
- **YAN, H. ; H. FRIELINGHAUS et al.** Thermoreversible lysozyme hydrogels: properties and an insight into the gelation pathway. *Soft Matter,* 2008, vol. 4 (6), 1313-1325
- **YAN, H. ; A. SAIANI et al.** Thermoreversible Protein Hydrogel as Cell Scaffold. *Biomacromolecules,* 2006, vol. 7 (10), 2776-2782
- **YANLIAN, Y. ; K. ULUNG et al.** Designer self-assembling peptide nanomaterials. *Nano Today,* 2009, vol. 4 (2), 193-210
- Designer self-assembling peptide nanofiber scaffolds for study of 3-D cell biology and beyond. **ZHANG, S.** Advances in Cancer Research. Elsevier Academic Press Inc, 2008, vol. 99, 335